# EUROPÄISCHE PATENTSCHRIFT

(11) **EP 0 837 938 B1**
(45) Veröffentlichungstag und Bekanntmachung des Hinweises auf die Patenterteilung: **08.08.2012**
(21) Anmeldenummer: 96925740.1
(22) Anmeldetag: 12.07.1996
(51) Int. Cl.: C12N 15/12, C07K 14/495, A61K 38/18

(54) **VERWENDUNG VON MP52 ODER MP121 ZUR BEHANDLUNG UND PRÄVENTION VON ERKRANKUNGEN DES NERVENSYSTEMS**
USE OF MP52 OR MP121 FOR TREATING AND PREVENTING DISEASES OF THE NERVOUS SYSTEM
UTILISATION DE MP52 OU DE MP121 POUR LA THERAPIE ET LA PROPHYLAXIE DE MALADIES DU SYSTEME NERVEUX

(30) Priorität: 12.07.1995 DE 19525416
(43) Veröffentlichungstag der Anmeldung: 29.04.1998
(73) Patentinhaber: BIOPHARM GESELLSCHAFT ZUR BIOTECHNOLOGISCHEN ENTWICKLUNG VON PHARMAKA mbH, 69115 Heidelberg (DE)
(72) Erfinder: HÖTTEN, Gertrud, 69245 Bammental (DE); POHL, Jens, 76707 Hambrücken (DE); BECHTOLD, Rolf, 69126 Heidelberg (DE); PAULISTA, Michael, 69181 Leimen (DE); UNSICKER, Klaus, 69118 Heidelberg (DE)
(74) Vertreter: Böhm, Brigitte
(86) Internationale Anmeldenummer: PCT/EP1996/003065
(87) Internationale Veröffentlichungsnummer: WO 1997/003188

(56) Entgegenhaltungen:
- WO-A-93/16099
- WO-A-95/04819
- WO-A-96/01316
- KRIEGLSTEIN K ET AL: "Transforming growth factor-beta promotes survival of midbrain dopaminergic neurons and protects them against N-methyl-4-phenylpyridinium ion toxicity", NEUROSCIENCE, NEW YORK, NY, US, vol. 63, no. 4, 1 December 1994 (1994-12-01), pages 1189-1196, XP024383780, ISSN: 0306-4522, DOI: 10.1016/0306-4522(94)90583-5 [retrieved on 1994-12-01]

### SEQUENZPROTOKOLL

### (1) ALLGEMEINE INFORMATION:

(i) ANMELDER:
   (A) NAME: Biopharm Gesellschaft zur biotechnologischen Entwicklung von Pharmaka mbH
   (B) STRASSE: Czernyring 22
   (C) ORT: Heidelberg
   (E) LAND: DE
   (F) POSTLEITZAHL: 69115
(ii) ANMELDETITEL: Verwendung von MP52 oder/und MP121 zur Behandlung und Prävention von Erkrankungen des Nervensystems
(iii) ANZAHL DER SEQUENZEN: 4
(iv) COMPUTER-LESBARE FORM:
   (A) DATENTRÄGER: Floppy disk
   (B) COMPUTER: IBM PC compatible
   (C) BETRIEBSSYSTEM: PC-DOS/MS-DOS
   (D) SOFTWARE: PatentIn Release #1.0, Version #1.25 (EPA)

### (2) INFORMATION ZU SEQ ID NO: 1:

(i) SEQUENZ CHARAKTERISTIKA:
   (A) LÄNGE: 501 Aminosäuren
   (B) ART: Aminosäure
   (D) TOPOLOGIE: linear
(ii) ART DES MOLEKÜLS: Protein
(xi) SEQUENZBESCHREIBUNG:SEQ ID NO: 1:
   Met Arg Leu Pro Lys Leu Leu Thr Phe Leu Leu Trp Tyr Leu Ala Trp
    1               5                  10                  15
   Leu Asp Leu Glu Phe Ile Cys Thr Val Leu Gly Ala Pro Asp Leu Gly
               20                  25                  30
   Gln Arg Pro Gln Gly Thr Arg Pro Gly Leu Ala Lys Ala Glu Ala Lys
           35                  40                  45
   Glu Arg Pro Pro Leu Ala Arg Asn Val Phe Arg Pro Gly Gly His Ser
       50                  55                  60
   Tyr Gly Gly Gly Ala Thr Asn Ala Asn Ala Arg Ala Lys Gly Gly Thr
   65                  70                  75                  80
   Gly Gln Thr Gly Gly Leu Thr Gln Pro Lys Lys Asp Glu Pro Lys Lys
                   85                  90                  95
   Leu Pro Pro Arg Pro Gly Gly Pro Glu Pro Lys Pro Gly His Pro Pro
               100                 105                 110
   Gln Thr Arg Gln Ala Thr Ala Arg Thr Val Thr Pro Lys Gly Gln Leu
           115                 120                 125
   Pro Gly Gly Lys Ala Pro Pro Lys Ala Gly Ser Val Pro Ser Ser Phe
       130                 135                 140
   Leu Leu Lys Lys Ala Arg Glu Pro Gly Pro Pro Arg Glu Pro Lys Glu
   145                 150                 155                 160
   Pro Phe Arg Pro Pro Pro Ile Thr Pro His Glu Tyr Met Leu Ser Leu
                   165                 170                 175
   Tyr Arg Thr Leu Ser Asp Ala Asp Arg Lys Gly Gly Asn Ser Ser Val
               180                 185                 190
   Lys Leu Glu Ala Gly Leu Ala Asn Thr Ile Thr Ser Phe Ile Asp Lys
           195                 200                 205
   Gly Gln Asp Asp Arg Gly Pro Val Val Arg Lys Gln Arg Tyr Val Phe
       210                 215                 220
   Asp Ile Ser Ala Leu Glu Lys Asp Gly Leu Leu Gly Ala Glu Leu Arg
   225                 230                 235                 240
   Ile Leu Arg Lys Lys Pro Ser Asp Thr Ala Lys Pro Ala Ala Pro Gly
                   245                 250                 255
   Gly Gly Arg Ala Ala Gln Leu Lys Leu Ser Ser Cys Pro Ser Gly Arg
               260                 265                 270
   Gln Pro Ala Ser Leu Leu Asp Val Arg Ser Val Pro Gly Leu Asp Gly
           275                 280                 285
   Ser Gly Trp Glu Val Phe Asp Ile Trp Lys Leu Phe Arg Asn Phe Lys
       290                 295                 300
   Asn Ser Ala Gln Leu Cys Leu Glu Leu Glu Ala Trp Glu Arg Gly Arg
   305                 310                 315                 320
   Ala Val Asp Leu Arg Gly Leu Gly Phe Asp Arg Ala Ala Arg Gln Val
                   325                 330                 335
   His Glu Lys Ala Leu Phe Leu Val Phe Gly Arg Thr Lys Lys Arg Asp
               340                 345                 350
   Leu Phe Phe Asn Glu Ile Lys Ala Arg Ser Gly Gln Asp Asp Lys Thr
           355                 360                 365
   Val Tyr Glu Tyr Leu Phe Ser Gln Arg Arg Lys Arg Arg Ala Pro Leu
       370                 375                 380
   Ala Thr Arg Gln Gly Lys Arg Pro Ser Lys Asn Leu Lys Ala Arg Cys
   385                 390                 395                 400
   Ser Arg Lys Ala Leu His Val Asn Phe Lys Asp Met Gly Trp Asp Asp
                   405                 410                 415
   Trp Ile Ile Ala Pro Leu Glu Tyr Glu Ala Phe His Cys Glu Gly Leu
               420                 425                 430
   Cys Glu Phe Pro Leu Arg Ser His Leu Glu Pro Thr Asn His Ala Val
           435                 440                 445
   Ile Gln Thr Leu Met Asn Ser Met Asp Pro Glu Ser Thr Pro Pro Thr
   450 455 460
   Cys Cys Val Pro Thr Arg Leu Ser Pro Ile Ser Ile Leu Phe Ile Asp
   465 470 475 480
   Ser Ala Asn Asn Val Val Tyr Lys Gln Tyr Glu Asp Met Val Val Glu
   485 490 495
   Ser Cys Gly Cys Arg
   500

### (2) INFORMATION ZU SEQ ID NO: 2:

(i) SEQUENZ CHARAKTERISTIKA:
   (A) LÄNGE: 2703 Basenpaare
   (B) ART: Nukleinsäure
   (C) STRANGFORM: beides
   (D) TOPOLOGIE: linear
(ii) ART DES MOLEKÜLS: DNS
(vi) URSPRÜNGLICHE HERKUNFT:
   (A) ORGANISMUS: Homo sapiens
(ix) MERKMALE:
   (A) NAME/SCHLÜSSEL: CDS
   (B) LAGE: 640..2142
(xi) SEQUENZBESCHREIBUNG: SEQ ID NO: 2:
   CCATGGCCTC GAAAGGGCAG CGGTGATTTT TTTCACATAA ATATATCGCA CTTAAATGAG     60
   TTTAGACAGC ATGACATCAG AGAGTAATTA AATTGGTTTG GGTTGGAATT CCGTTTCCAA    120
   TTCCTGAGTT CAGGTTTGTA AAAGATTTTT CTGAGCACCT GCAGGCCTGT GAGTGTGTGT    180
   GTGTGTGTGT GTGTGTGTGT GTGTGTGTGA AGTATTTTCA CTGGAAAGGA TTCAAAACTA    240
   GGGGGAAAAA AAAACTGGAG CACACAGGCA GCATTACGCC ATTCTTCCTT CTTGGAAAAA    300
   TCCCTCAGCC TTATACAAGC CTCCTTCAAG CCCTCAGTCA GTTGTGCAGG AGAAAGGGGG    360
   CGGTTGGCTT TCTCCTTTCA AGAACGAGTT ATTTTCAGCT GCTGACTGGA GACGGTGCAC    420
   GTCTGGATAC GAGAGCATTT CCACTATGGG ACTGGATACA AACACACACC CGGCAGACTT    480
   CAAGAGTCTC AGACTGAGGA GAAAGCCTTT CCTTCTGCTG CTACTGCTGC TGCCGCTGCT    540
   TTTGAAAGTC CACTCCTTTC ATGGTTTTTC CTGCCAAACC AGAGGCACCT TTGCTGCTGC    600
   CGCTGTTCTC TTTGGTGTCA TTCAGCGGCT GGCCAGAGG ATG AGA CTC CCC AAA    654
                                             Met Arg Leu Pro Lys
                                              1               5
   CTC CTC ACT TTC TTG CTT TGG TAC CTG GCT TGG CTG GAC CTG GAA TTC    702
   Leu Leu Thr Phe Leu Leu Trp Tyr Leu Ala Trp Leu Asp Leu Glu Phe
                    10                  15                  20
   ATC TGC ACT GTG TTG GGT GCC CCT GAC TTG GGC CAG AGA CCC CAG GGG    750
   Ile Cys Thr Val Leu Gly Ala Pro Asp Leu Gly Gln Arg Pro Gln Gly
                25                  30                  35
   ACC AGG CCA GGA TTG GCC AAA GCA GAG GCC AAG GAG AGG CCC CCC CTG    798
   Thr Arg Pro Gly Leu Ala Lys Ala Glu Ala Lys Glu Arg Pro Pro Leu
            40                  45                  50
   GCC CGG AAC GTC TTC AGG CCA GGG GGT CAC AGC TAT GGT GGG GGG GCC    846
   Ala Arg Asn Val Phe Arg Pro Gly Gly His Ser Tyr Gly Gly Gly Ala
        55                  60                  65
   ACC AAT GCC AAT GCC AGG GCA AAG GGA GGC ACC GGG CAG ACA GGA GGC 894
   Thr Asn Ala Asn Ala Arg Ala Lys Gly Gly Thr Gly Gln Thr Gly Gly
   70 75 80 85
   CTG ACA CAG CCC AAG AAG GAT GAA CCC AAA AAG CTG CCC CCC AGA CCG 942
   Leu Thr Gln Pro Lys Lys Asp Glu Pro Lys Lys Leu Pro Pro Arg Pro
   90 95 100
   GGC GGC CCT GAA CCC AAG CCA GGA CAC CCT CCC CAA ACA AGG CAG GCT 990
   Gly Gly Pro Glu Pro Lys Pro Gly His Pro Pro Gln Thr Arg Gln Ala
   105 110 115
   ACA GCC CGG ACT GTG ACC CCA AAA GGA CAG CTT CCC GGA GGC AAG GCA 1038
   Thr Ala Arg Thr Val Thr Pro Lys Gly Gln Leu Pro Gly Gly Lys Ala
   120 125 130
   CCC CCA AAA GCA GGA TCT GTC CCC AGC TCC TTC CTG CTG AAG AAG GCC 1086
   Pro Pro Lys Ala Gly Ser Val Pro Ser Ser Phe Leu Leu Lys Lys Ala
   135 140 145
   AGG GAG CCC GGG CCC CCA CGA GAG CCC AAG GAG CCG TTT CGC CCA CCC 1134
   Arg Glu Pro Gly Pro Pro Arg Glu Pro Lys Glu Pro Phe Arg Pro Pro
   150 155 160 165
   CCC ATC ACA CCC CAC GAG TAC ATG CTC TCG CTG TAC AGG ACG CTG TCC 1182
   Pro Ile Thr Pro His Glu Tyr Met Leu Ser Leu Tyr Arg Thr Leu Ser
   170 175 180
   GAT GCT GAC AGA AAG GGA GGC AAC AGC AGC GTG AAG TTG GAG GCT GGC 1230
   Asp Ala Asp Arg Lys Gly Gly Asn Ser Ser Val Lys Leu Glu Ala Gly
   185 190 195
   CTG GCC AAC ACC ATC ACC AGC TTT ATT GAC AAA GGG CAA GAT GAC CGA 1278
   Leu Ala Asn Thr Ile Thr Ser Phe Ile Asp Lys Gly Gln Asp Asp Arg
   200 205 210
   GGT CCC GTG GTC AGG AAG CAG AGG TAC GTG TTT GAC ATT AGT GCC CTG 1326
   Gly Pro Val Val Arg Lys Gln Arg Tyr Val Phe Asp Ile Ser Ala Leu
   215 220 225
   GAG AAG GAT GGG CTG CTG GGG GCC GAG CTG CGG ATC TTG CGG AAG AAG 1374
   Glu Lys Asp Gly Leu Leu Gly Ala Glu Leu Arg Ile Leu Arg Lys Lys
   230 235 240 245
   CCC TCG GAC ACG GCC AAG CCA GCG GCC CCC GGA GGC GGG CGG GCT GCC 1422
   Pro Ser Asp Thr Ala Lys Pro Ala Ala Pro Gly Gly Gly Arg Ala Ala
   250 255 260
   CAG CTG AAG CTG TCC AGC TGC CCC AGC GGC CGG CAG CCG GCC TCC TTG 1470
   Gln Leu Lys Leu Ser Ser Cys Pro Ser Gly Arg Gln Pro Ala Ser Leu
   265 270 275
   CTG GAT GTG CGC TCC GTG CCA GGC CTG GAC GGA TCT GGC TGG GAG GTG 1518
   Leu Asp Val Arg Ser Val Pro Gly Leu Asp Gly Ser Gly Trp Glu Val
   280 285 290
   TTC GAC ATC TGG AAG CTC TTC CGA AAC TTT AAG AAC TCG GCC CAG CTG 1566
   Phe Asp Ile Trp Lys Leu Phe Arg Asn Phe Lys Asn Ser Ala Gln Leu
   295 300 305
   TGC CTG GAG CTG GAG GCC TGG GAA CGG GGC AGG GCC GTG GAC CTC CGT 1614
   Cys Leu Glu Leu Glu Ala Trp Glu Arg Gly Arg Ala Val Asp Leu Arg
   310                 315                 320                 325
   GGC CTG GGC TTC GAC CGC GCC GCC CGG CAG GTC CAC GAG AAG GCC CTG      1662
   Gly Leu Gly Phe Asp Arg Ala Ala Arg Gln Val His Glu Lys Ala Leu
                   330                 335                 340
   TTC CTG GTG TTT GGC CGC ACC AAG AAA CGG GAC CTG TTC TTT AAT GAG      1710
   Phe Leu Val Phe Gly Arg Thr Lys Lys Arg Asp Leu Phe Phe Asn Glu
               345                 350                 355
   ATT AAG GCC CGC TCT GGC CAG GAC GAT AAG ACC GTG TAT GAG TAC CTG      1758
   Ile Lys Ala Arg Ser Gly Gln Asp Asp Lys Thr Val Tyr Glu Tyr Leu
           360                 365                 370
   TTC AGC CAG CGG CGA AAA CGG CGG GCC CCA CTG GCC ACT CGC CAG GGC      1806
   Phe Ser Gln Arg Arg Lys Arg Arg Ala Pro Leu Ala Thr Arg Gln Gly
       375                 380                 385
   AAG CGA CCC AGC AAG AAC CTT AAG GCT CGC TGC AGT CGG AAG GCA CTG      1854
   Lys Arg Pro Ser Lys Asn Leu Lys Ala Arg Cys Ser Arg Lys Ala Leu
   390                 395                 400                 405
   CAT GTC AAC TTC AAG GAC ATG GGC TGG GAC GAC TGG ATC ATC GCA CCC      1902
   His Val Asn Phe Lys Asp Met Gly Trp Asp Asp Trp Ile Ile Ala Pro
                   410                 415                 420
   CTT GAG TAC GAG GCT TTC CAC TGC GAG GGG CTG TGC GAG TTC CCA TTG      1950
   Leu Glu Tyr Glu Ala Phe His Cys Glu Gly Leu Cys Glu Phe Pro Leu
               425                 430                 435
   CGC TCC CAC CTG GAG CCC ACG AAT CAT GCA GTC ATC CAG ACC CTG ATG      1998
   Arg Ser His Leu Glu Pro Thr Asn His Ala Val Ile Gln Thr Leu Met
           440                 445                 450
   AAC TCC ATG GAC CCC GAG TCC ACA CCA CCC ACC TGC TGT GTG CCC ACG      2046
   Asn Ser Met Asp Pro Glu Ser Thr Pro Pro Thr Cys Cys Val Pro Thr
       455                 460                 465
   CGG CTG AGT CCC ATC AGC ATC CTC TTC ATT GAC TCT GCC AAC AAC GTG      2094
   Arg Leu Ser Pro Ile Ser Ile Leu Phe Ile Asp Ser Ala Asn Asn Val
   470                 475                 480                 485
   GTG TAT AAG CAG TAT GAG GAC ATG GTC GTG GAG TCG TGT GGC TGC AGG      2142
   Val Tyr Lys Gln Tyr Glu Asp Met Val Val Glu Ser Cys Gly Cys Arg
                   490                 495                 500
   TAGCAGCACT GGCCCTCTGT CTTCCTGGGT GGCACATCCC AAGAGCCCCT TCCTGCACTC    2202
   CTGGAATCAC AGAGGGGTCA GGAAGCTGTG GCAGGAGCAT CTACACAGCT TGGGTGAAAG    2262
   GGGATTCCAA TAAGCTTGCT CGCTCTCTGA GTGTGACTTG GGCTAAAGGC CCCCTTTTAT    2322
   CCACAAGTTC CCCTGGCTGA GGATTGCTGC CCGTCTGCTG ATGTGACCAG TGGCAGGCAC    2382
   AGGTCCAGGG AGACAGACTC TGAATGGGAC TGAGTCCCAG GAAACAGTGC TTTCCGATGA    2442
   GACTCAGCCC ACCATTTCTC CTCACCTGGG CCTTCTCAGC CTCTGGACTC TCCTAAGCAC    2502
   CTCTCAGGAG AGCCACAGGT GCCACTGCCT CCTCAAATCA CATTTGTGCC TGGTGACTTC    2562
   CTGTCCCTGG GACAGTTGAG AAGCTGACTG GGCAAGAGTG GGAGAGAAGA GGAGAGGGCT    2622
   TGGATAGAGT TGAGGAGTGT GAGGCTGTTA GACTGTTAGA TTTAAATGTA TATTGATGAG    2682
   ATAAAAAGCA AAACTGTGCC T                                               2703

### (2) ANGABEN ZU SEQ ID NO: 3:

(i) SEQUENZKENNZEICHEN:
   (A) LÄNGE: 352 Aminosäuren
   (B) ART: Aminosäure
   (C) STRANGFORM:
   (D) TOPOLOGIE: linear
(ii) ART DES MOLEKÜLS: Protein
(vi) URSPRÜNGLICHE HERKUNFT:
   (A) ORGANISMUS: Homo sapiens
(xi) SEQUENZBESCHREIBUNG: SEQ ID NO: 3:
   Met Thr Ser Ser Leu Leu Leu Ala Phe Leu Leu Leu Ala Pro Thr Thr
   1 5 10 15
   Val Ala Thr Pro Arg Ala Gly Gly Gln Cys Pro Ala Cys Gly Gly Pro
   20 25 30
   Thr Leu Glu Leu Glu Ser Gln Arg Glu Leu Leu Leu Asp Leu Ala Lys
   35 40 45
   Arg Ser Ile Leu Asp Lys Leu His Leu Thr Gln Arg Pro Thr Leu Asn
   50 55 60
   Arg Pro Val Ser Arg Ala Ala Leu Arg Thr Ala Leu Gln His Leu His
   65 70 75 80
   Gly Val Pro Gln Gly Ala Leu Leu Glu Asp Asn Arg Glu Gln Glu Cys
   85 90 95
   Glu Ile Ile Ser Phe Ala Glu Thr Gly Leu Ser Thr Ile Asn Gln Thr
   100 105 110
   Arg Leu Asp Phe His Phe Ser Ser Asp Arg Thr Ala Gly Asp Arg Glu
   115 120 125
   Val Gln Gln Ala Ser Leu Met Phe Phe Val Gln Leu Pro Ser Asn Thr
   130 135 140
   Thr Trp Thr Leu Lys Val Arg Val Leu Val Leu Gly Pro His Asn Thr
   145 150 155 160
   Asn Leu Thr Leu Ala Thr Gln Tyr Leu Leu Glu Val Asp Ala Ser Gly
   165 170 175
   Trp His Gln Leu Pro Leu Gly Pro Glu Ala Gln Ala Ala Cys Ser Gln
   180 185 190
   Gly His Leu Thr Leu Glu Leu Val Leu Glu Gly Gln Val Ala Gln Ser
   195 200 205
   Ser Val Ile Leu Gly Gly Ala Ala His Arg Pro Phe Val Ala Ala Arg
   210 215 220
   Val Arg Val Gly Gly Lys His Gln Ile His Arg Arg Gly Ile Asp Cys
   225 230 235 240
   Gln Gly Gly Ser Arg Met Cys Cys Arg Gln Glu Phe Phe Val Asp Phe
   245 250 255
   Arg Glu Ile Gly Trp His Asp Trp Ile Ile Gln Pro Glu Gly Tyr Ala
                   260                 265                 270
   Met Asn Phe Cys Ile Gly Gln Cys Pro Leu His Ile Ala Gly Met Pro
               275                 280                 285
   Gly Ile Ala Ala Ser Phe His Thr Ala Val Leu Asn Leu Leu Lys Ala
       290                 295                 300
   Asn Thr Ala Ala Gly Thr Thr Gly Gly Gly Ser Cys Cys Val Pro Thr
   305                 310                 315                 320
   Ala Arg Arg Pro Leu Ser Leu Leu Tyr Tyr Asp Arg Asp Ser Asn Ile
                       325                 330                 335
   Val Lys Thr Asp Ile Pro Asp Met Val Val Glu Ala Cys Gly Cys Ser
                   340                 345                 350

### (2) ANGABEN ZU SEQ ID NO: 4:

(i) SEQUENZKENNZEICHEN:
   (A) LÄNGE: 352 Aminosäuren
   (B) ART: Aminosäure
   (C) STRANGFORM:
   (D) TOPOLOGIE: linear
(ii) ART DES MOLEKÜLS: Protein
(vi) URSPRÜNGLICHE HERKUNFT:
   (A) ORGANISMUS: Mouse
(xi) SEQUENZBESCHREIBUNG: SEQ ID NO: 4:
   Met Ala Ser Ser Leu Leu Leu Ala Leu Leu Phe Leu Thr Pro Thr Thr
   1               5                   10                  15
   Val Val Asn Pro Lys Thr Glu Gly Pro Cys Pro Ala Cys Trp Gly Ala
               20                  25                  30
   Ile Phe Asp Leu Glu Ser Gln Arg Glu Leu Leu Leu Asp Leu Ala Lys
           35                  40                  45
   Lys Ser Ile Leu Asp Lys Leu His Leu Ser Gln Arg Pro Ile Leu Ser
       50                  55                  60
   Arg Pro Val Ser Arg Gly Ala Leu Lys Thr Ala Leu Gln Arg Leu Arg
   65                  70                  75                  80
   Gly Pro Arg Arg Glu Thr Leu Leu Glu His Asp Gln Arg Gln Glu Glu
                   85                  90                  95
   Tyr Glu Ile Ile Ser Phe Ala Asp Thr Asp Leu Ser Ser Ile Asn Gln
               100                 105                 110
   Thr Arg Leu Glu Phe His Phe Ser Gly Arg Met Ala Ser Gly Met Glu
           115                 120                 125
   Val Arg Gln Thr Arg Phe Met Phe Phe Val Gln Phe Pro His Asn Ala
       130                 135                 140
   Thr Gln Thr Met Asn Ile Arg Val Leu Val Leu Arg Pro Tyr Asp Thr
   145                 150                 155                 160
   Asn Leu Thr Leu Thr Ser Gln Tyr Val Val Gln Val Asn Ala Ser Gly
                   165                 170                 175
   Trp Tyr Gln Leu Leu Leu Gly Pro Glu Ala Gln Ala Ala Cys Ser Gln
               180                 185                 190
   Gly His Leu Thr Leu Glu Leu Val Pro Glu Ser Gln Val Ala His Ser
           195                 200                 205
   Ser Leu Ile Leu Gly Trp Phe Ser His Arg Pro Phe Val Ala Ala Gln
       210                 215                 220
   Val Arg Val Glu Gly Lys His Arg Val Arg Arg Arg Gly Ile Asp Cys
   225                 230                 235                 240
   Gln Gly Gly Ser Arg Met Cys Cys Arg Gln Glu Phe Phe Val Asp Phe
                   245                 250                 255
   Arg Glu Ile Gly Trp Asn Asp Trp Ile Ile Gln Pro Glu Gly Tyr Ala
                   260                 265                 270
   Met Asn Phe Cys Thr Gly Gln Cys Pro Leu His Val Ala Gly Met Pro
               275                 280                 285
   Gly Ile Ser Ala Ser Phe His Thr Ala Val Leu Asn Leu Leu Lys Ala
           290                 295                 300
   Asn Ala Ala Ala Gly Thr Thr Gly Arg Gly Ser Cys Cys Val Pro Thr
   305                 310                 315                 320
   Ser Arg Arg Pro Leu Ser Leu Leu Tyr Tyr Asp Arg Asp Ser Asn Ile
                   325                 330                 335
   Val Lys Thr Asp Ile Pro Asp Met Val Val Glu Ala Cys Gly Cys Ser
                   340                 345                 350

## Beschreibung

Die vorliegende Erfindung betrifft die Verwendung von MP52 oder/und MP121, zweier Wachstums- oder/und Differenzierungsfaktoren der TGF-β-Superfamilie zur Behandlung und Prävention von Erkrankungen des Nervensystems oder/und zur Behandlung neuropathologischer Situationen, die durch Alterung des Nervensystems verursacht sind. Darüber hinaus betrifft die Erfindung Arzneimittel zur Behandlung oder Prävention obiger Indikationen, welche MP52 oder/und MP121 enthalten.

Viele Wachstumsfaktoren aus der TGF-β-Superfamilie (Kingsley, Genes & Development 8, 133-146 (1994) und die darin zitierte Literatur) sind für einen weiten Bereich medizinischer Behandlungsmethoden und Anwendungen, die insbesondere Wundheilung und Gewebewiederherstellung betreffen, relevant. Zu diesen zählen insbesondere Mitglieder der TGF-β (Transforming Growth Factor, siehe z.B. Roberts und Sporn, Handbook of Experimental Pharmacology 95 (1990), S. 419-472, eds. Sporn und Roberts), der BMP (Bone Morphogenetic Protein, siehe z.B. Rosen und Thies, Growth Factors in Perinatal Development (1993), S. 39-58, eds. Tsang, Lemons und Balistreri) und der Inhibin/Aktivin (siehe z.B. Vale et al., The Physiology of Reproduction, Second Edition, (1994), S. 1861-1878, eds. Knobil und Neill) Familie. Obwohl die Mitglieder dieser Familie im reifen Anteil hohe Aminosäurehomologien, insbesondere zumeist 7 konservierte Cysteine aufweisen, zeigen sie erhebliche Variationen in ihren genauen Funktionen. Die TGF-β-ähnlichen Proteine gehören zu einer strukturellen Superfamilie, die alle ein Cystine Knot Motif aufweisen (Cell, Vol. 73 (1993), S. 421-424). Weitere Mitglieder dieser Superfamilie sind Proteine aus der NGF (Nerve Growth Factor)-/Neurotrophin-Familie und PDGF (Platelet Derived Growth Factor)-Familie. Häufig zeigen einzelne Wachstumsfaktoren mehrere Funktionen gleichzeitig, so daß ihre Anwendung bei verschiedenen medizinischen Indikationen von Interesse ist.

Einige dieser multifunktionellen Proteine zeigen neben Funktionen wie z.B. Regulation der Proliferation und Differenzierung bei vielen Zelltypen (Roberts und Sporn, Handbook of Experimental Pharmacology 95 (1990), S. 419-472, eds. Sporn und Roberts; Sakurai et al., J. Biol. Chem. 269 (1994), S. 14118-14122) auch überlebensfördernde Effekte bei Neuronen. So wurden z.B. für TGF-β trophische Effekte auf embryonalen motorischen und sensorischen Neuronen in vitro nachgewiesen (Martinou et al., Devl. Brain Res. 52, S. 175-181 (1990) und Chalazonitis et al., Dev. Biol. 152, S. 121-132 (1992)). Weiterhin wurden für die Proteine TGF-β-1, -2, -3, Aktivin A und GDNF (Glial cell line-derived neurotrophic factor), ein Protein, das strukturelle Ähnlichkeiten zu TGF-β-Superfamilienmitgliedern aufweist, überlebensfördernde Effekte auf dopaminerge Neurone des Mittelhirns nachgewiesen, wobei dieser Effekt nicht über Astrozyten vermittelt wurde (Krieglstein et al., EMBO J. 14, S. 736-742 (1995)).

WO 93/16099, WO 95/04819 und WO96/01316 offenbaren die DNA- und Protein-Sequenzen von TGF-β ähnlichen Proteinen, insbesondere von MP52 und MP121. In WO 95/04819 wird für MP52 ein knorpel- und knocheninduzierendes Potential offenbart.

Das Auftreten von Proteinen der TGF-β-Superfamilie in unterschiedlichen Gewebestufen und Entwicklungsstufen ist in Übereinstimmung mit Unterschieden hinsichtlich genauer Funktionen, sowie Zielorte, Lebensdauer, Erfordernisse für Hilfsfaktoren, erforderliche zelluläre physiologische Umgebung und/oder Beständigkeit gegen Abbau.

Die der vorliegenden Erfindung zu Grunde liegende Aufgabe besteht darin, ein Protein bereitzustellen, das eine Behandlung oder Prävention von Krankheiten des Nervensystems ermöglicht. Von Interesse sind die Behandlung von Störungen oder Verlusten nervöser Funktionen. Diese können hervorgerufen werden durch akute pathologische Zustände wie bei zerebrovaskulären, entzündlichen, infektiösen, stoffwechselartigen Mängeln oder/und Mängeln hervorgerufen durch toxische Einflüsse, Verletzungen, Tumorwachstum oder operative Eingriffe. Weiterhin können Störungen oder Verluste nervöser Funktionen verursacht sein durch chronische pathologische Zustände wie vorzugsweise neurodegenerative Erkrankungen. Neuropathologische Situationen werden häufig auch durch Alterung des Nervensystems verursacht.

Diese Aufgabe wird gelöst durch die Verwendung von biologisch aktivem MP52 oder/und MP121 zur Behandlung oder/und Prävention von Erkrankungen des Nervensystems oder/und zur Behandlung neuropathologischer Situationen, die durch Alterung des Nervensystems verursacht sind.

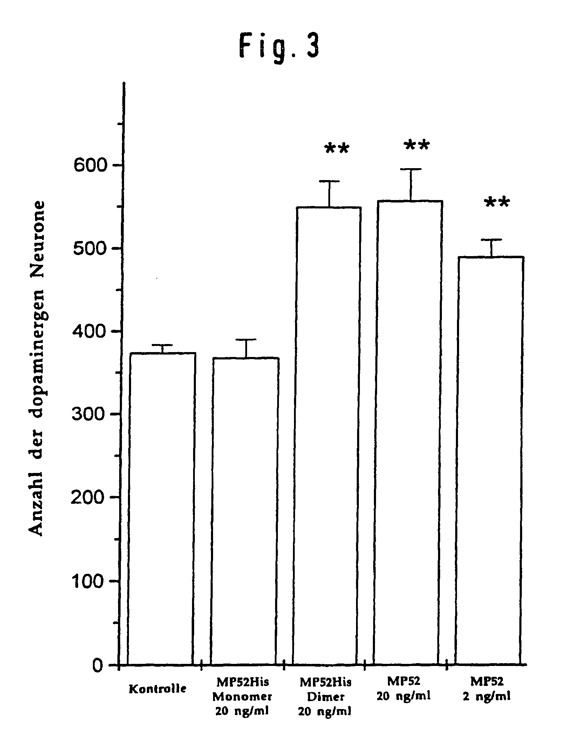

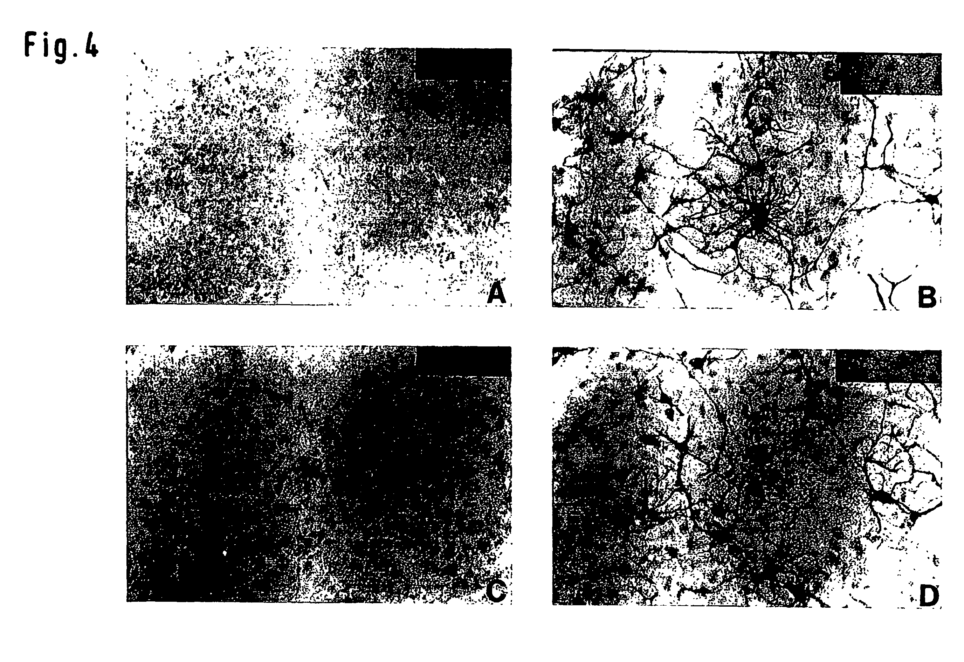

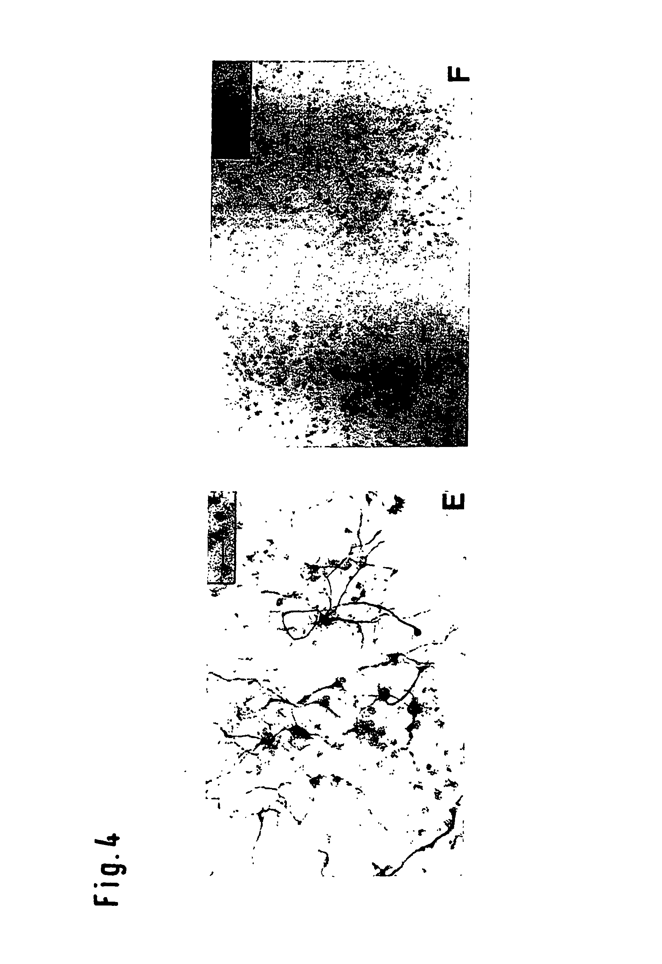

Mit der vorliegenden Erfindung konnte gezeigt werden, daß MP52 einen positiven Einfluß auf das Überleben von dopaminergen Neuronen aufweist (siehe Figur 3). Dieser Einfluß wird jedoch abweichend von TGF-βs und Aktivin A zumindest teilweise über die mit Nervenzellen assoziierten Astrozyten vermittelt (siehe Figur 4). Somit ist MP52 nützlich bei der Behandlung oder Prävention von Krankheiten des Nervensystems, insbesondere Erkrankungen, die das Gehirn betreffen. Von besonderem Interesse sind hierbei neurodegenerative Erkrankungen wie z.B. die Parkinson'sche Krankheit, möglicherweise auch Krankheiten wie Alzheimer oder Huntington Chorea. Desweiteren gelingt es unter Verwendung von MP52 das Überleben von Neuronen zu fördern und damit eine Aufrechterhaltung nervöser Funktionen zu bewirken. Alle Anwendungsmöglichkeiten bestehen sowohl bei akuten als auch bei chronisch pathologischen Zuständen, auch die Vorbeugemaßnahmen. Als akute pathologische Zustände sind dabei vorzugsweise zerebrovaskuläre, entzündliche, infektiöse, stoffwechselartige Mängelerscheinungen oder/und Mängel, hervorgerufen durch toxische Einflüsse, Verletzungen, Tumorwachstum oder operative Eingriffe zu nennen.

Ein im Rahmen der Erfindung behandelbarer chronischer pathologischer Zustand ist z.B. eine neurodegenerative Erkrankung. Mit der vorliegenden Erfindung konnte weiterhin gezeigt werden, daß MP52 einen stimulierenden Einfluß auch auf Neurone der Retina hat. Während der Entwicklung des visuellen Systems wandern die Axone aus den Ganglionzellen der Retina zu speziellen Regionen im Gehirn. Es konnte durch mehrere Gruppen gezeigt werden, daß lösliche Faktoren, die aus visuellen Bereichen des Gehirns isoliert wurden, die Ganglionzellen in der Retina stimulieren können (Nurcombe, V. und Bennett, M.R., Exp. Brain Res. 44, 249-258 (1981), Hyndman, A.G., Adler, R., Dev. Neurosci. 5, 40-53 (1982), Turner, J.E. et al., Dev. Brain Res. 6, 77-83 (1983), Carri, N.G. und Ebendal, T., Dev. Brain Res. 6, 219-229 (1983)). Die Bildung von Nervenfaserbündeln, welche wahrscheinlich optische Axone sind, die von Ganglionzellen der Retina ausgehen, hängt von neurotrophen Faktoren ab.

Versuche mit MP52 zeigten, daß dieses Protein auch als ein neurotropher Faktor in diesem System wirken kann.

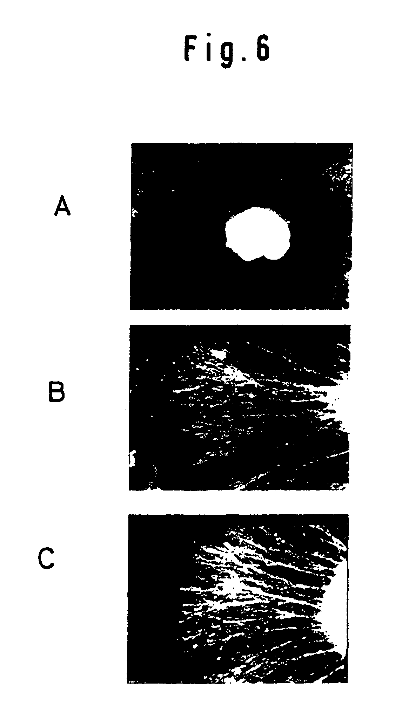

So konnte an frisch isolierten Gewebekulturen von embryonaler Retina aus Hühnern gezeigt werden, daß MP52 das Auswachsen von Nervenfasern signifikant fördert (siehe hierzu Figur 6 und Tabelle 1).

Während dieser Experimente konnte auch gezeigt werden, daß weitere Mitglieder der TGF-ß-Familie ebenfalls eine stimulierende Wirkung haben. Dazu zählt insbesondere auch der MP121 (WO93/1609 und WO96/01316) der eine etwa gleich starke Wirkung wie der MP52 aufweist (siehe hierzu Figur 6 und Tabelle 2).

Die Aktivitäten von MP52 und MP121 können für die Heilung von Erkrankungen am Auge, insbesondere der Retina und des Sehnervs verwendet werden. Hervorzuheben sind hier insbesondere auch die Behandlung von Verletzungen der neuronalen Schicht der Retina und des Sehnervs. Derartige Verletzungen könnten z.B. durch Unfälle, Entzündungen oder Durchblutungsstörungen hervorgerufen werden. Anwendungen bei Netzhauttransplantationen sind ebenfalls von Vorteil. Weiterhin sollte aber auch eine Heilung oder Linderung von Verletzungen an anderen Hirnnerven von Bedeutung sein. Hervorzuheben ist hier z.B. der Trigeminus (Nervus Trigeminus), der auch Teile des Auges innerviert. So können Mitglieder der TGF-ß-Familie, insbesondere der MP52 und der MP121, auch Anwendung bei Hornhauttransplantationen finden. Das Anwachsen der Hornhaut wird auch durch die nervale Versorgung beeinflußt. Denkbar ist ein Einsatz auch bei nur segmentalen Schäden der Hornhaut, wie sie z.B. bei Herpesinfektionen im Auge auftreten.

Insbesondere die Anwendung bei degenerierenden Erkrankungen der Augenoberfläche sind hervorzuheben.

In einer bevorzugten Ausführungsform der vorliegenden Erfindung verwendet man als biologisch aktiven MP52
(a) den reifen Anteil und gegebenenfalls weitere funktionelle Anteile der in SEQ ID NO.1 gezeigten Proteinsequenz;
(b) Teile des reifen Proteins, die im wesentlichen dieselbe Aktivität aufweisen;
(c) reife Proteine mit verändertem N-Terminus, welche im wesentlichen dieselbe Aktivität aufweisen;
(d) ein reifes Protein oder Teile davon, welches aufgrund seiner Abstammung aus anderen Wirbeltieren Abweichung in der Aminosäuresequenz aufweist, jedoch im wesentlichen dieselbe Aktivität beibehält.

In einer anderen bevorzugten Ausführungsform verwendet man als biologisch aktiven MP121
(a) den reifen Anteil und gegebenenfalls weitere funktionelle Anteile der in SEQ ID NO. 3 oder 4 gezeigten Proteinsequenz
(b) Teile des reifen Proteins, die im wesentlichen dieselbe Aktivität aufweisen;
(c) reife Proteine mit verändertem N-Terminus, welche im wesentlichen dieselbe Aktivität aufweisen;
(d) ein reifes Protein oder Teile davon, welches aufgrund seiner Abstammung aus anderen Wirbeltieren Abweichung in der Aminosäuresequenz aufweist, jedoch im wesentlichen dieselbe Aktivität beibehält.

Andere Merkmale und Vorteile der Erfindung gehen aus der Beschreibung der bevorzugten Ausführungsformen und den Figuren hervor. Die Sequenzprotokolle und Figuren sind im folgenden kurz beschrieben.

SEQ ID NO.1 zeigt die vollständige Aminosäuresequenz des Präproproteins des humanen TGF-ß-Proteins MP52, die aus der in SEQ ID NO.2 gezeigten Nukleotidsequenz abgeleitet wurde und in WO 95/04819 bereits offenbart ist. Der Beginn des reifen Proteins liegt vorzugsweise im Bereich der Aminosäuren 361-400, besonders bevorzugt bei Aminosäure 382.

SEQ ID NO.2: zeigt die vollständige Nukleotidsequenz der für das TGF-ß-Protein MP52 kodierenden DNA, wie sie bereits in WO 95/04819 offenbart ist. Das ATG-Startcodon beginnt mit Nukleotid 640. Der Start des vollständigen reifen Proteins beginnt besonders bevorzugt hinter Nukleotid 1782. Das Stopcodon beginnt mit Nukleotid 2143.

SEQ ID NO.3 zeigt die vollständige Aminosäuresequenz des Präproproteins des humanen TGF-ß-Proteins MP121, die bereits in der WO96/01316 offenbart ist. Der Beginn des reifen Proteins liegt vorzugsweise im Bereich der Aminosäuren 217-240, besonders bevorzugt bei Aminosäure 236 oder 237, am meisten bevorzugt bei Aminosäure 237.

SEQ ID NO.4 zeigt die vollständige Aminosäuresequenz des Präproproteins des TGF-ß-Proteins MP121 aus der Maus, die ebenfalls in der WO96/01316 offenbart ist. Der Beginn des reifen Proteins liegt in Analogie zum humanen Protein im Bereich der Aminosäuren 217 - 240, der Beginn des bevorzugten reifen Proteins bei Aminosäure 236 oder 237.
Figur 1 zeigt ein silbergefärbtes Gel mit im prokaryontischen System exprimiertem, reifem MP52 mit vorangestelltem Methionin sowie reifem MP52 mit einem Histidin-Anhang am N-Terminus vor und nach dem Rückfalten.
Figur 2 zeigt ein Chromatogramm zur Trennung von dimeren und monomerem reifen MP52 mit verändertem N-Terminus nach dem Rückfalten.
Figur 3 zeigt einen positiven Einfluß auf das Überleben von dopaminergen Neuronen durch Behandlung mit teilgereinigten MP52 aus einem eukaryontem Expressionssystem sowie gereinigtem zurückgefaltetem, reifen MP52 mit verändertem N-Terminus aus einem prokaryontischem Expressionssystem.
Figur 4 zeigt, daß der überlebensfördernde Effekt von MP52 auf dopaminerge Neurone zumindest zum Teil auf eine Erhöhung der Anzahl von Astrozyten zurückzuführen ist.
Figur 5 zeigt einen Western blot mit Kaninchen-Antikörpern gegen MP121, der mit Hilfe des Vaccina-Viren-Expressionssystems in HepG2-Zellen synthetisiert wurde.
Figur 6 zeigt den stimulierenden Einfluß von gereinigtem MP52 und MP121 auf das Auswachsen von Nervenfasern aus der embryonalen Retina.

Im Rahmen der vorliegenden Erfindung umfaßt der reife Anteil des MP52-Proteins Aminosäure 382 bis Aminosäure 501. Bei MP121 umfaßt der reife Anteil vorzugsweise Aminosäure 237 bis Aminosäure 352 der SEQ ID NO.3 bzw. der SEQ ID NO.4. Darüber hinaus sind im Rahmen der Erfindung gegebenenfalls aber auch kürzere oder längere funktionelle Teilbereiche des Gesamtproteins eingeschlossen, die im wesentlichen gleiche biologische Aktivität aufweisen, insbesondere Teilbereiche die mindestens den Bereich der sieben konservierten Cysteine umfassen. In der vorliegenden Erfindung konnte unter anderem gezeigt werden, daß Modifikationen am N-Terminus des reifen Proteins die Aktivität nicht wesentlich beeinflussen.

Umfaßt sind auch MP52- bzw. MP121-Proteine, die aus anderen Wirbeltieren isoliert sind, da diese im wesentlichen die gleichen Aktivitäten aufweisen.

Andererseits können Proteine neben dem reifen Anteil von MP52 oder MP121 auch noch funktionelle Signal- oder/und Propeptidanteile von anderen Proteinen, z.B. von Proteinen mit Cystine Knot Motif (Cell, Vol. 73 (1993) S. 421-424) und insbesondere von anderen Proteinen der TGF-ß Superfamilie, z.B. den oben genannten TGF- oder BMP- oder Aktivin/Inhibin-Proteinen, insbesondere auch MP121 bzw. MP52 umfassen. Die entsprechenden Nukleotidsequenzen sind aus den oben genannten Referenzen und der darin zitierten Literatur und/oder der EMBL-Datenbank oder GenBank zu entnehmen, auf deren Offenbarung hiermit Bezug genommen wird. Wichtig ist hierbei, daß der richtige Leserahmen für das reife Protein erhalten bleibt. Je nachdem, in welchen Wirtszellen die Expression stattfindet, könnte das Vorhandensein einer anderen Signalsequenz oder/und eines anderen Propeptidanteils die Expression positiv beeinflussen. Der Austausch von Propeptidanteilen durch entsprechende Anteile anderer Proteine ist z.B. bei Mol. Endocrinol. 5 (1991), S. 149-155 und Proc. Natl. Acad. Sci. USA 90 (1993), S. 2905-2909 beschrieben.

Eine weitere bevorzugte Ausführungsform der Erfindung ist die Verwendung von Fusionsproteinen, die funktionelle Derivate bzw. Anteile von MP52 bzw. MP121 gemäß der obigen Definition und wie vorzugsweise in SEQ ID NO.1 bzw. 3 oder 4 gezeigt, insbesondere funktionelle Anteile des reifen Proteins aufweisen und darüber hinaus Anteile eines anderen Proteins. Das andere Protein kann hierbei wiederum ein Protein mit "Cystine Knot Motif" sein, das vorzugsweise auch zur TGF-ß Superfamilie gehört, wie z.B. auch Kombination von MP 52 mit MP121. Es können jedoch auch Anteile eines komplett anderen Proteins vorhanden sein, z.B. Rezeptor-bindende Domänen von Proteinen, welche dem ursprünglichen MP52- bzw. MP121-Protein eine andere Spezifität verleihen. Eine wiederum weitere bevorzugte Ausführungsform der Erfindung ist die Verwendung heterodimerer Proteine aus einem Monomer eines biologisch aktiven MP52 oder MP121 gemäß obiger Definition und einem Monomer eines Proteins aus der Superfamilie von Proteinen mit "Cystine Knot Motif", bevorzugt eines Mitgliedes der TGF-ß Superfamilie. Ähnliche heterodimere Proteine sind z.B. in der WO 93/09229, EP 0 626 451 A2 und J. Biol. Chem. 265 (1990), S. 13198-13205 beschrieben. Die Merkmale des Proteins können in Abhängigkeit von der Bildung von Homo- oder Heterodimeren variieren und für therapeutische Anwendungen relevant sein.

Die in SEQ ID NO.2 gezeigte DNA-Sequenz, Teile davon oder eine Sequenz, die für erfindungsgemäße chimäre Proteine kodiert, können zur Produktion von MP52 verwendet werden. Die Expression kann in eukaryonten und prokaryonten Wirtszellen erfolgen. Geeignete Expressionssysteme sind dem Fachmann bekannt und es ist leicht feststellbar, welcher minimale Anteil von SEQ ID NO.2 erforderlich ist, um ein exprimiertes Protein zu erhalten, das in den angeführten Versuchen Aktivität zeigt. Zu den Expressionssystemen zählen auch Virensysteme wie z.B. das Baculovirus- oder das Vaccinia-Virus-System. Zur Herstellung einer ausreichenden Menge der gereinigten erfindungsgemäßen Proteine aus der Wirtszelle oder/und dem Zellkulturüberstand zum Einsatz bei der medizinischen Behandlung kann ein Bakterium, wie etwa E.coli oder Bacillus, ein Pilz, wie etwa Hefe, eine Pflanzenzelle, wie etwa Tabak oder Arabidopsis oder eine tierische Wirbeltierzellinie, wie etwa CHO, HuTK-, NIH-3T3, COS oder Mo-Zellinien oder eine Insektenzellinie wie etwa von Spodoptera frugiperda (SF9) oder Trichoplusia ni (TN368) verwendet werden. Unter Ausnutzung rekombinanter Insektenviren wie z.B. Bombyx mori Nuclear Polyhedrosis Virus oder Baculoviren ist eine Expression auch in Insektenlarven wie etwa Bombyx mori oder Spodoptera frugiperda möglich. Ein solches System ist z.B. beschrieben bei Ishida et al. (J. Biochem. 115 (1994), S. 279-285). Bei der Herstellung in Bakterien kann das erfindungsgemäße Protein in Form von Einschlußkörpern (inclusion bodies) vorliegen. Diese Einschlußkörper werden nach an sich bekannten Methoden renaturiert, um das Protein in einer aktiven Form zu erhalten. Es konnte innerhalb der Erfindung gezeigt werden, daß eine Renaturierung zum dimeren MP52 für die Aktivität notwendig ist, da monomeres MP52 keine Aktivität zeigt (siehe Figur 3). Ähnliches gilt für die Herstellung von MP121, die in der WO96/01316 detailliert beschrieben ist.

Zur Herstellung von heterodimeren Proteinen mit anderen Mitgliedern der "Cystine Knot Familie" werden beide Proteinmonomere entweder in derselben Zelle oder getrennt exprimiert, wobei auch eine gemeinsame Renaturierung bei anfallenden Formen von inclusion bodies geeignet erscheint. Bei Coexpression in derselben Zelle sind wiederum insbesondere auch virale Systeme, wie z.B. das Baculoviren-System oder das Vaccinia-Viren System geeignet. Die Herstellung und Reinigung von heterodimeren Proteinen ist prinzipiell dem Fachmann bekannt und z.B. in der WO 93/09229 und der EP 0 626 451 A2 beschrieben. Heterodimere können von Homodimeren z.B. dadurch getrennt werden, daß nacheinander Affinitätssäulen mit Antikörpern spezifisch für jeweils ein Monomer eingesetzt werden.

Die Herstellung von chimären Proteinen mit anderen Proteinanteilen erfordert eine entsprechende Veränderung auf DNA-Ebene, die dem Fachmann geläufig ist und von ihm durchgeführt werden kann (EMBO J. 10 (1991), S.2105-2110; Cell 69 (1992) S.329-341; J. Neurosci. 39 (1994), S.195-210).

In einer weiteren bevorzugten Ausführungsform verabreicht man zusätzlich zu biologisch aktivem MP52 oder/und MP121 in einer der oben erwähnten Formen natürlich vorkommende Ganglioside, deren Derivate, Salze oder künstliche Analoga. Desweiteren ist es bevorzugt, zusätzlich einen Wachstumsfaktor zu verabreichen. Dabei handelt es sich vorzugsweise um ein Protein aus der Superfamilie der Proteine mit "Cystine Knot Motif", wobei es sich vorzugsweise um ein Protein aus der TGF-ß-Superfamilie, der NGF-/Neurotrophin-Familie oder der PDGF-Familie handelt. Besonders bevorzugt sind Wachstumsfaktoren, die zur NGF-/Neurotrophin-Familie zählen wie z.B. die Wachstumsfaktoren NGF, BDNF, NT-3 oder NT4/5. (siehe auch Guidebook to Cytokines and their Receptors, Nicos A., Nicola, A Sambrook and Tooze Publication, Oxford University Press, 1994, Seite 140-143 und die darin zitierte Literatur). Andere bevorzugte Wachstumsfaktoren sind FGF, EGF und Glial Growth Factor. Derartige Kombinationen können zu synergistischen Wirkungen führen, wie es z.B. für Mitglieder der TGF-ß-Superfamilie gezeigt ist (Ogawa et al., J. Biol. Chem. 267 (1992), 14233-14237 oder US 5413989).

Soweit dies nötig oder vorteilhaft ist, ist es für den Fachmann selbstverständlich, desweiteren in der Pharmazie übliche Träger-, Hilfs-, Verdünnungs- oder Füllstoffe zuzusetzen.

Die Verabreichung einer MP52- oder/und MP121-haltigen Zusammensetzung wird zweckmäßigerweise so vorgenommen, daß eine größtmögliche Wirkung erzielt wird, wobei insbesondere ein Einsatz nahe am Zielort von Vorteil sein kann. Die Verabreichung kann u.a. intrazerebral, oral, durch Injektion, durch Inhalation oder als lokale äußerliche Anwendung erfolgen.

Ein weiterer Gegenstand der vorliegenden Erfindung ist die Verwendung von biologisch aktivem MP52 oder/und MP121 zur Herstellung eines Arzneimittels zur Behandlung oder/und Prävention von Erkrankungen des Nervensystems oder/und zur Behandlung neuropathologischer Situationen, die durch Alterung des Nervensystems verursacht sind. Bevorzugte Ausführungsformen und Anwendungsmöglichkeiten entsprechen hierbei den Ausführungsformen, die bereits für den vorgenannten Gegenstand der vorliegenden Erfindung detailliert beschrieben wurden. Ein weiterer Gegenstand der vorliegenden Erfindung ist ein Fusionsprotein, das zumindest Teile des reifen Anteils der in SEQ ID NO.1 gezeigten Sequenz von MP52 sowie mindestens Teile eines anderen Proteins aus der Superfamilie der Proteine mit "Cystine Knot Motif" enthält.

Ein derartiges erfindungsgemäßes Fusionsprotein enthält vorzugsweise
(a) den gesamten reifen Proteinanteil aus SEQ ID NO.1 sowie gegebenenfalls weitere funktionelle Anteile der in SEQ ID NO.1 gezeigten Proteinsequenz;
(b) den reifen Anteil der in SEQ ID NO.1 gezeigten Proteinsequenz, jedoch mit verändertem N-Terminus, bevorzugt einem vorangestellten Methionin; oder
(c) den reifen Anteil der in SEQ ID NO.1 gezeigten Proteinsequenz oder Teile davon, welche aufgrund ihrer Herkunft aus anderen Wirbeltieren Abweichungen aufweist, jedoch im wesentlichen die gleiche Aktivität beibehält.

Die Herstellung solcher Fusionsproteine ist bereits oben beschrieben ebenso beispielhafte geeignete Proteine, aus denen der nicht MP52-Proteinteil abgeleitet werden kann.

Wiederum ein weiterer Gegenstand der vorliegenden Erfindung ist ein heterodimeres Protein, das mindestens einen Teil der in SEQ ID NO.1 gezeigten Sequenz des reifen MP52-Proteins als ein Monomer sowie ein zweites Monomer eines anderen Proteins aus der Superfamilie der Proteine mit "Cystine Knot Motif" enthält. Auch hier ist es wiederum bevorzugt, daß der MP52-Anteil (das MP52-Monomer)
(a) den gesamten reifen Proteinanteil aus SEQ ID NO.1 sowie gegebenenfalls weitere funktionelle Anteile der in SEQ ID NO.1 gezeigten Proteinsequenz;
(b) den reifen Anteil der in SEQ ID NO.1 gezeigten Proteinsequenz, jedoch mit verändertem N-Terminus, bevorzugt einem vorangestellten Methionin; oder
(c) den reifen Anteil der in SEQ ID NO.1 gezeigten Proteinsequenz oder Teile davon, welche aufgrund ihrer Herkunft aus anderen Wirbeltieren Abweichungen aufweist, jedoch im wesentlichen die gleiche Aktivität beibehält,
enthält.

Die Herstellung von heterodimeren Proteinen sowie Proteine, die sich als Zweitmonomer eignen, sind wiederum bereits oben in der Beschreibung erwähnt.

Noch ein weiterer Gegenstand der vorliegenden Erfindung sind Arzneimittel zur Behandlung und Prävention von Erkrankungen des Nervensystems oder/und zur Behandlung neuropathologischer Situationen, die durch Alterung des Nervensystems verursacht sind und die eine biologisch wirksame Menge von MP52 oder/und MP121 als Wirkstoff enthalten. Das erfindungsgemäße Arzneimittel enthält als biologisch aktiven MP52 vorzugsweise
(a) den reifen Anteil und gegebenenfalls weitere funktionelle Anteile der in SEQ ID NO.1 gezeigten Proteinsequenz;
(b) Teile des reifen Proteins, die im wesentlichen dieselbe Aktivität aufweisen;
(c) reife Proteine mit verändertem N-Terminus, welche im wesentlichen dieselbe Aktivität aufweisen;
(d) ein reifes Protein oder Teile davon, welches aufgrund seiner Abstammung aus anderen Wirbeltieren Abweichung in der Aminosäuresequenz aufweist, jedoch im wesentlichen dieselbe Aktivität beibehält.

In einer zweiten bevorzugten Form enthält das erfindungsgemäße Arzneimittel als biologisch aktiven MP121
(a) den reifen Anteil und gegebenenfalls weitere funktionelle Anteile der in SEQ ID NO.3 oder 4 gezeigten Proteinsequenz;
(b) Teile des reifen Proteins, die im wesentlichen dieselbe Aktivität aufweisen;
(c) reife Proteine mit verändertem N-Terminus, welche im wesentlichen dieselbe Aktivität aufweisen;
(d) ein reifes Protein oder Teile davon, welches aufgrund seiner Abstammung aus anderen Wirbeltieren Abweichung in der Aminosäuresequenz aufweist, jedoch im wesentlichen dieselbe Aktivität beibehält.

Die Therapiemöglichkeiten des Proteins können abhängig von der Bildung von Homodimeren oder Heterodimeren mit anderen Proteinen mit "Cystine Knot Motif" und insbesondere TGF-ß- oder NGF-Proteinen sowie durch Verwendung chimärer Proteine variieren. Solche Strukturen können sich ebenfalls für klinische Anwendungen geeignet erweisen und bilden daher ebenfalls einen Gegenstand der vorliegenden Anmeldung.

Umfaßt sind somit auch pharmazeutische Zusammensetzungen, die erfindungsgemäße heterodimere Proteine und/oder Fusionsproteine enthalten. Von Vorteil kann bei einer pharmazeutischen Zusammensetzung auch die Kombination von MP52 oder MP121 mit anderen Proteinen der TGF-ß Superfamilie wie z.B. GDNF, den TGF-ßs, den BMPs und den Aktivinen oder mit Proteinen der NGF-/Neurotrophin-Familie wie z.B. dem NGF, den Neurotrophinen wie z.B. NT-3, -4/5 oder BDNF (Brain-Derived Neurotrophic Factor) oder auch Wachstumsfaktoren wie FGF (Fibroblast Growth Factor), EGF (Epidermal Growth Factor), Glial Growth Factor, PDGF (Platelet-Derived Growth Factor) sein. Solche Kombinationen sind ebenfalls Gegenstand der Anmeldung.

Gegebenenfalls umfaßt eine erfindungsgemäße Zusammensetzung neben den Wirkstoffen pharmazeutisch akzeptablen Träger-, Hilfs-, Verdünnungs- oder/und Füllstoffe.

Bevorzugt kann eine solche Zusammensetzung auch natürlich vorkommende Ganglioside, deren Derivate, Salze oder künstlichen Analoga umfassen.

Zur Behandlung oder Prävention von Nervenerkrankungen können MP52 oder MP121 injiziert, oral, nicht-oral, intrazerebral, durch Inhalation, als lokale, äußerliche Anwendung oder durch jegliche andere pharmazeutisch übliche Methode verabreicht werden. Die Dosis liegt im Bereich von 0,1 bis 1000 µg/kg Körpergewicht.

Denkbar ist eine Applikation von biologisch aktivem MP52 oder/und MP121 bzw. von erfindungsgemäßen heterodimeren Proteinen und/oder chimären Proteinen z.B. aber auch über implantierte nicht menschliche embryonale Stammzellen, die zuvor durch Transfektion geeigneter DNA Elemente zu einer konstitutiven Expression der erfindungsgemäßen Proteine, insbesondere von MP52 oder MP121 gebracht wurden. Weiterhin möglich ist eine Bereitstellung der erfindungsgemäßen Proteine, insbesondere von biologisch aktivem MP52 oder MP121, durch bestimmte virale Systeme. Somit ist es möglich, geeignete Vektoren mit der erfindungsgemäßen DNA Sequenz in vitro oder in vivo in Patientenzellen zu transfizieren oder die Vektoren in vitro in Zellen zu transfizieren und diese dann einem Patienten zu implantieren.

Weiterhin ist die Anwendung dieser pharmazeutischen Zusammensetzung nicht auf Menschen beschränkt, sondern kann auch Tiere, insbesondere Haustiere umfassen.

Generell können mit den erfindungsgemäß verwendeten Proteinen Krankheiten des Nervensystems behandelt werden, die in irgendeiner Form mit der Expression von MP52 bzw. MP121 zusammenhängen oder auf MP52 oder MP121 auf irgendeine Weise ansprechen, einerseits durch die Erhöhung der Menge bzw. der Aktivität an vorhandenem MP52 oder MP121, auf der anderen Seite auch durch Unterdrückung der MP52- bzw. MP121-Aktivität. Eine Unterdrückung der MP52- bzw. MP121-Aktivität kann durch Hemmung der Transkription und/oder der Translation z.B. durch dem Fachmann bekannte Antisense-Nukleinsäuren erfolgen. Eine andere Möglichkeit ist die Bindung von Molekülen an MP52- oder MP121-Rezeptoren, die im Gegensatz zu MP52 bzw. MP121 keine Signalweiterleitung auslösen. Es sind daher im Rahmen der Erfindung auch die Rezeptoren für MP52 oder MP121 an Zellen von Interesse. Zum Auffinden von Rezeptoren können zunächst verschiedene Zellinien auf ihr Bindungsverhalten von radioaktiv markiertem MP52 bzw. MP121 (¹²⁵J-MP52 oder ¹²⁵J-MP121) mit anschließenden cross-linking getestet werden. Von Zellen, die MP52 bzw. MP121 binden, kann nachfolgend eine cDNA Bibliothek in einem Expressionsvektor (z.B. erhältlich bei InVitrogen) angelegt werden. Zellen die mit Rezeptor cDNA transfiziert wurden, können dann über die Bindung von radioaktiv markiertem MP52 bzw. MP121 selektiert werden. Dies sind dem Fachmann bekannte Methoden, wie sie z.B. für die Isolierung von Aktivin- (Mathews, L.S. & Vale, W.W. Cell 65 (1991) S.973-982) und TGF-ß-Rezeptoren Typ II (Lin et al. Cell 68 (1992) S.775-785) verwendet wurden. Es ist in Analogie zu den bekannten Aktivin-, TGF-ß- und BMP- Rezeptoren zu vermuten, daß es sich bei den Rezeptor für MP52 und MP121 ebenfalls um einen in diese Familie gehörenden Rezeptorkomplex handelt, so daß zum Auffinden von Teilen des heteromeren Komplexes weitere dem Fachmann bekannte Methoden wie z.B. PCR mit degenerierten Oligonukleotiden verwendet werden können. Diese Methode ist z.B. auch bei dem Aktivin-Rezeptor Typ I, TGF-ß-Rezeptoren Typ I und BMP-Rezeptoren Typ I angewendet worden (Tsuchida et al. (1993) Proc. Natl. Acad. Sci. USA 90, 11242-11246; Attisano et al. (1993) Cell 75, 671-680; Franzén et al. (1993) Cell 75, 681-692; ten Dijke et al. (1994) J. Biol. Chem. 269, 16985-16988; Koenig et al. (1994) Mol. Cell. Biol. 14, 5961-5974).

Die Erfindung soll durch die folgenden Beispiele veranschaulicht werden.

### Beispiel 1:

### Eukaryontische Expression und Reinigung von MP52

Für die Expression von MP52 wurden Vaccinia Viren gewählt, deren Verwendung ausführlich und für den Fachmann nacharbeitbar in den Current Protocols in Molecular Biology (Ausubel et al., Greene Publishing Associates and Wiley-Interscience, Wiley & Sons (1989-1995)) im folgenden abgekürzt mit CP unter Chapter 16 Unit 16.15-16.18 beschrieben ist. Die cDNA mit dem gesamten kodierenden Bereich für MP52 wurde in den Vektor pBP₁ kloniert. Das resultierende Plasmid (pBP1MP52s) wurde bei der DSM (Hinterlegungsnummer 9217) am 24. Mai 1994 hinterlegt und für die Herstellung von rekombinanten Vaccinia Viren wie in WO 95/04819 offenbart eingesetzt. Die Expression von MP52 in 143B Zellen (HuTk-, ATCC CRL 8303) nach Infektion mit rekombinanten Vaccinia Viren erfolgte wie offenbart in WO 95/04819. Die Teilreinigung von MP52 erfolgte, wie in derselben Offenbarung beschrieben, über eine Heparin Säule (HiTrap^{™}, Pharmacia #17-0407-01) und einer anschließenden Reversed Phase HPLC (C₃-Säule, Aquapore RP300, Applied Biosystems, Partikelgröße: 7 µm, Porengröße: 300 Ä). Die Fraktionen mit MP52 wurden gepoolt, lyophilisiert und bei -80°C aufbewahrt.

### Beispiel 2:

### Prokaryontische Expression, Reinigung und Rückfaltung zum aktiven MP52

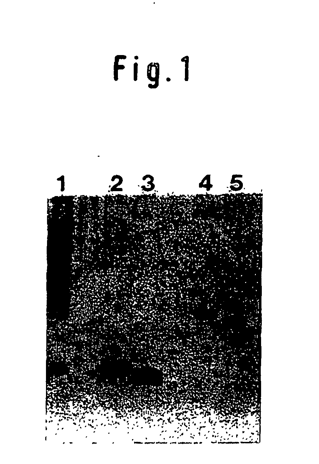

Die mögliche Expression von MP52 in E.coli ist bereits in WO 93/16099 und WO 95/04819 offenbart, die Expression von reifem MP52 auch mit angehängten Histidinen am N-Terminus ist ebenfalls offenbart (WO 95/04819). Die zusätzlichen Histidine erleichtern die Aufreinigung des Proteins durch Bindung an Metallchelat-Säulen. Nach Aufreinigung kann das in E.coli als Monomer exprimierte reife MP52 Protein dann zu einem Dimer zurückgefallen werden. Der größte Teil des reifen Anteils von MP52 (Aminosäure 383 bis 501 in SEQ ID NO.2) mit 10 zusätzlichen Aminosäuren einschließlich 6 Histidinen am N-Terminus (MHHHHHHKLI) wurde in dem prokaryontischen Vektor pBP2 exprimiert. Dieser Vektor ist ein pBR322 Derivat mit Ampicillinresistenz, der zusätzlich den T7 Promoter aus dem pBluescript II SK Plasmid (Stratagene) enthält. Weiterhin enthält der Vektor nach dem T7-Promoter eine ribosomale Bindestelle und ein Startcodon als Teil einer Nde I Restriktionsschnittstelle gefolgt von 6 Codons für Histidin. Hinter mehreren Restriktionsschnittstellen (Hind III, Eco RI, Xho I, Bam HI, Sma I und Apa I) für die Insertion von Inserts sowie Stopcodons in allen drei Leserahmen folgt ein Terminator (Tø). Das Plasmid pBP2MP52His wurde am 2. Juni 1995 bei der DSM (Hinterlegungsnummer: DSM 10028) hinterlegt. Derselbe Vektor wurde unter Ausnutzung der Nde I Restriktionsschnittstelle zur Expression des reifen MP52 (Aminosäure 382 bis 501 in SEQ ID NO.1) mit nur einem zusätzlichen Methionin am N-Terminus verwendet. Das Plasmid pBP2MP52m wurde am 2. Juni 1995 bei der DSM (Hinterlegungsnummer: DSM 10029) hinterlegt. Die Expression von MP52 Protein mit (MP52His) oder ohne (MP52m) Histidin Anhang kann durch gleichzeitige Bereitstellung von T7-RNA Polymerase erfolgen. Die T7-RNA Polymerase kann über verschiedene Methoden bereitgestellt werden, wie z.B. ein zweites Plasmid mit einem Gen für T7 RNA Polymerase oder durch Infektion mit Phagen, die für die T7 RNA Polymerase kodieren oder aber durch spezielle Bakterienstämme, die das Gen für T7 RNA Polymerase integriert haben. Unter Verwendung des Bakterienstammes BL21(DE3)pLysS (Novagen, #69451-1) und Induktion der T7 RNA Polymerase nach Herstellerangaben mit IPTG, wird das MP52 Protein mit und ohne His-Tag in Einschlußkörpern gebildet aus denen die Proteine nach Standardmethoden isoliert werden können. Aufgrund des His-Tag kann MP52His Protein über metallchelatbildende Säulen wie z.B. beschrieben in Hochuli et al. (BIO/Technology Vol. 6, 1321-1325 (1988)) gereinigt werden. MP52His und MP52m wurden weiter über eine Reversed Phase HPLC gereinigt. Es wurde eine Reversed Phase Säule (Nucleosil 300-7C4 von Macherey-Nagel, Art. 715023) mit einer Flußrate von 2 ml/min und einem Acetonitrilgradienten in 0.1 % TFA von 0 bis 90% in 100 min verwendet. Unter diesen Bedingungen beginnt die Elution von monomerem MP52His und MP52m bei ca. 35% Acetonitril. Der Nachweis, daß es sich um MP52 Protein handelt, wurde jeweils über Western blot Analyse mit MP52 spezifischen Antikörpern geführt. MP52m (121 Aminosäuren) bzw. MP52His (129 Aminosäuren) zeigen in SDS-Polyacrylamid-Gelen (15%) ein apparentes Molekulargewicht von ca. 14 kD (theoretisches Molekulargewicht: 13.7 kD) bzw. 15 kD (theoretisches Molekulargewicht: 14.8 kD) wie es nach Silberfärbung in Figur 1 gezeigt ist.

Um biologisch aktives Material zu erhalten, kann das in E.coli exprimierte und aufgereinigte monomere MP52 zu einem dimeren MP52 zurückgefaltet werden. Dies kann nach dem Fachmann bekannten Methoden, wie z.B. beschrieben von Jaenicke, R. & Rudolph, R. (Protein structure, ed. Creighton, T.E., IRL Press, chapter 9 (1989)), erfolgen. Da für jedes Protein die Bedingungen zum Rückfalten im einzelnen variieren, wurden die Bedingungen für die Proteine MP52His und MP52m in Hinblick auf die Solubilisierung sowie pH-Werte, Temperatur und Redoxsysteme während der Renaturierung getestet. Für die Solubilisierung der gereinigten und lyophilisierten MP52 Proteine zeigten sich typische Reagenzien wie Harnstoff und Guanidiniumchlorid als geeignet. Vorzugsweise erfolgte die Solubilisierung über 2 Stunden bei Raumtemperatur in Solubilisierungspuffer (6 M Guanidiniumchlorid, 10 mM Tris, 150 mM NaCl, 3 mM DTT pH 8.0) auf eine Endkonzentration von 2.6 mg MP52His oder MP52m pro ml. Das Solubilisat wurde dann zu Renaturierungspuffer vorzugsweise in einer Endkonzentration von 150 - 200 µg MP52His oder MP52m pro ml gegeben.

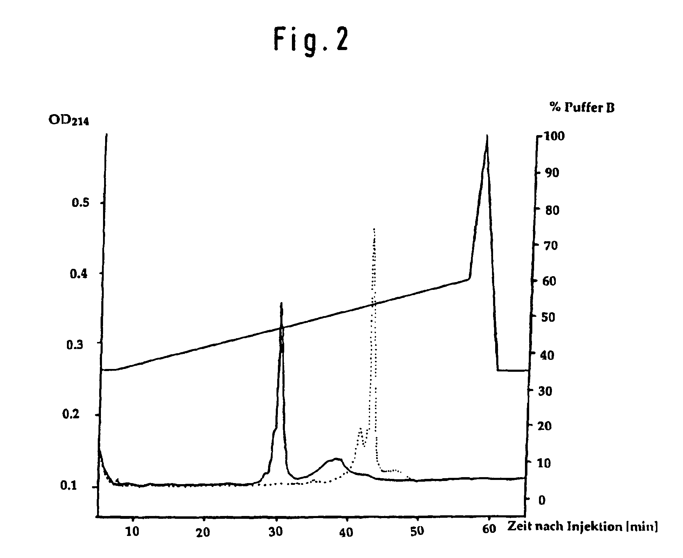

Für die Renaturierung zeigte sich, daß die Verwendung hoher pH Bereiche (pH 8 - 10) sich günstig auf das Zurückfalten zu aktiven MP52-Dimer Proteinen auswirkte. Dabei können gängige Puffersysteme wie Phosphat- oder Tris-Puffer mit 1-2 M NaCl und weiteren Zusätzen wie z.B. EDTA (2-10 mM) und Chaps (15 - 50 mM) verwendet werden. Es können in der Literatur beschriebene (z.B. Jaenicke, R. & Rudolph, R., Protein structure, ed. Creighton, T.E., IRL Press, chapter 9 (1989)), gängige Redoxsysteme wie z.B. oxidiertes und reduziertes Glutathion (z.B.: 1 mM GSSG, 2 mM GSH) eingesetzt werden. Das Zurückfalten kann effektiv im Bereich von 4°C bis Raumtemperatur über z.B. 48 Stunden durchgeführt werden. MP52 Proteine lassen sich unter solchen Bedingungen zu 50-90 % in die dimere Form überführen. Die genannten Bedingungen sind als beispielhaft zu betrachten und sind nicht limitierend. Es ist dem Fachmann möglich, durch Variation einzelner Bedingungen, MP52 mit ähnlichen Effizienzen in ein dimeres aktives Protein zu überführen. Die Analyse der rückgefalteten Proteine erfolgte über Reversed Phase HPLC sowie in silbergefärbten Gelen. Für die HPLC wurden die MP52 Proteine an eine Säule (Aquapore RP-300, 7 µm, Applied Biosystems) bei 35 % Puffer B (Puffer A: 0,1 % TFA in Wasser; Puffer B: 90 % Acetonitril, 0.1 % TFA) mit einer Flußrate von 0.2 ml/min gebunden. In einem Acetonitrilgradienten von 35 bis 60 % Puffer B über 50 min lassen sich die monomeren und dimeren MP52 Proteine voneinander trennen (siehe Figur 2). Der Anteil von rückgefaltetem MP52His bzw. MP52m wird auf ca. 70-90 % geschätzt. In 15 % Polyacrylamid Gelen läuft das dimere MP52m bei ungefähr 22 kD (theoretisches Molekulargewicht: 27,4 kD) und MP52His bei ungefähr 24 kD (theoretisches Molekulargewicht: 29,6 kD) abgeschätzt nach einem Molekulargewichtsmarker (siehe Figur 1).Der Histidin Anhang zeigt dabei keine signifikante Beeinflussung der Rückfaltungseffizienz. Testet man die Aktivität beider Proteine über die Bestimmung der alkalischen Phosphatase (ALP) Aktivität auf ROB-C26 Zellen, wie es z.B. in WO 95/04819 offenbart ist, so zeigt sich, daß MP52His trotz des veränderten N-Terminus aktiv ist, die Aktivität gegenüber MP52m aber leicht reduziert ist.

### Beispiel 3:

### Einfluß von MP52 auf dopaminerge Neurone

Zur Überprüfung des Einflusses von MP52 auf dopaminerge Neurone wurden Neurone vom Mittelhirnboden von 14 Tage alten Rattenembryonen (E14) nach einer Methode beschrieben bei Shimoda et al. (Brain Res. 586, 319-331 (1992)) isoliert. Die Vereinzelung und Kultivierung der Zellen erfolgte wie beschrieben bei Krieglstein et al. (Neuroscience 63, 1189-1196 (1994)). Die Zelldichte beträgt 200.000 Zellen/cm² auf Polyornithin/Laminin beschichteten Deckgläsern. Nach 24 Stunden Kultivierung und anschließend alle drei Tage wurden zwei Drittel des Mediums (500 µl) entfernt und ersetzt durch frisches Medium mit den entsprechenden Zusätzen. Das nach der Heparin-Sepharose und Reversed Phase HPLC lyophilisierte teilgereinigte MP52 wurde in 50% Acetonitril gelöst und dem Medium zugesetzt. Gleiches erfolgte mit zurückgefaltetem und gereinigtem MP52His. Die Endkonzentration von MP52 bzw. MP52His im Medium beträgt 20 ng/ml (Endkonzentration von Acetonitril ist 0.3 %). Als Kontrolle wurde eine vergleichbare Menge von gereinigtem monomerem MP52His in 50% Acetonitril gelöst und eingesetzt. Die Mediumkontrolle enthält ebenfalls 0.3 % Acetonitril. Nach acht Tagen wurden die Kulturen in 4% Paraformaldehyd für 10 min bei Raumtemperatur fixiert, die Zellen mit Aceton (10 min, -20°C) permeabilisiert und mit PBS (Phosphate buffered saline) gewaschen. Nach Behandlung mit 1% H₂O₂ in PBS, waschen und Blocken mit Pferdeserum erfolgte eine immuncytochemische Färbung. Die Tyrosinhydroxylase (TH) ist ein limitierendes Enzym bei der Biosynthese von Dopamin und anderen Katecholaminen, so daß TH in den vorliegenden Kulturen (Noradrenalin enthaltende Zellen werden nicht isoliert) als Marker für dopaminerge Neurone verwendet werden kann. TH wurde nachgewiesen über eine einstündige Inkubation bei 37°C mit einem Maus monoklonalen Antikörper gegen Ratten TH (1:200 verdünnt, Boehringer Mannheim) und nachfolgender Detektion mit dem Vectastain ABC kit (Vecto Labs). Die TH-positiven Zellen wurden in einer Fläche von 0.12 cm² ausgezählt. Für die immuncytochemische Färbung von GFAP (glial fibrillary acidic protein) wurden die fixierten Zellen mit Aceton (20 min, 20°C) permeabilisiert und mit PBS (Phosphate buffered saline) gewaschen. Nach Inkubation mit einem 1:200 verdünnten Maus monoklonalen Antikörper gegen GFAP (Sigma) folgte eine Inkubation mit einem Peroxidase gekoppelten Antikörper gegen den monoklonalen Maus Antikörper. Die Visualisierung erfolgte nach Standardmethoden durch Zugabe des Enzym Substrates DAB (Diaminobenzidin).

### Beispiel 4:

### Untersuchung zur Transkription von MP52 in Gehirn und Rückenmark

Zur Feststellung, ob MP52 in Gehirn und/oder Rückenmark transkribiert wird, wurde die total RNA aus Rückenmark von Ratten und Gesamtgehirn bzw. einzelnen Regionen des Gehirns aus Mäusen nach Standardmethoden isoliert und nach dem Fachmann bekannten Methoden in cDNA umgeschrieben. Die cDNA, die aus jeweils 100 ng RNA gewonnen wurde, wurde in eine PCR (Polymerase Chain Reaction) eingesetzt. Die für die Amplifikation eingesetzten Primer (CAACAGCAGCGTGAAGTTGGAG und ACTAATGTCAAACACGTACCTCTG) befinden sich bei humaner genomischer DNA auf unterschiedlichen Exonen, so daß genomische DNA von cDNA unterschieden werden kann. Als Kontrolle wurden 0,5 µg genomische Maus DNA eingesetzt, die ebenfalls kein PCR Fragment wie cDNA ergibt. Die PCR wurde über 30 Cyclen (94°C, 54°C, 72°C) in je 50 µl Reaktionsansatz (je 200 µM NTPs, 30 pmol jeden Primers, 16.6 mM (NH₄)₂SO₄, 67 mM Tris/HCL pH 8.8, 2 mM MgCl₂, 6,7 µM EDTA, 10 mM ß-Mercaptoethanol, 170 µg/ml BSA, 5 U AmpliTaq (Perkin Elmer #N8080160) durchgeführt. Ein Drittel der PCR Produkte wurden im 4% Agarose Gel aufgetrennt, im Southern blot auf Membranen transferiert und durch Hybridisierung mit einer MP52 Probe die Spezifität der PCR Fragmente nachgewiesen. Es konnte gezeigt werden, daß MP52 sowohl im Rückenmark als auch in einzelnen Gehirnregionen transkribiert ist.

### Beispiel 5:

### Eukaryontische Expression und Reinigung von MP121

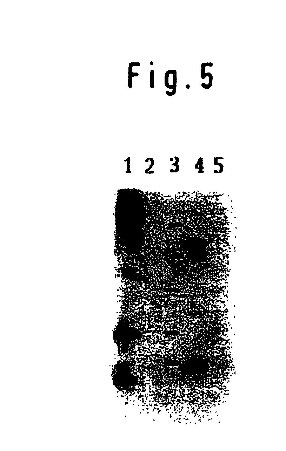

Für die Expression von MP121 wurden wie für MP52 in Beispiel 1 Vaccinia-Viren gewählt. Die cDNA mit dem gesamten codierenden Bereich für MP121 wurde in den Vektor pBP1 kloniert (das resultierende Plasmid pBPlMP121 wurde am 12.01.95 bei der DSM unter der Nummer 9665 hinterlegt) und zur Herstellung von rekombinanten Vaccinia-Viren eingesetzt. Bei Infektion von Zellen, wie z.B. NIH-3T3 Zellen (DSM ACC 59, swiss mouse embryo), mit den rekombinanten Viren, kommt es zur Expression von MP121. Die einzelnen Schritte sind detailliert in der WO96/01316 offenbart. Im Zuge der Expressionsversuche wurden auch andere Zellinien getestet. Dabei stellt sich überraschenderweise heraus, daß bei einigen Zellinien neben dem dimeren MP121 auch deutliche Mengen eines monomeren MP121 gebildet werden. Da dieses Monomer bei der Analyse im Polyacrylamidgel (mit anschließender Detektion durch Westernblot-Analyse) schneller läuft als das durch Reduktion des Dimers erhaltene Monomer, muß es sich um ein gefaltenes monomeres MP121, das eine globulärere Struktur aufweist, handeln. Als Beispiel ist in Figur 5 die Expression von MP121 in HepG2-Zellen (Hepatozelluläres Karzinom, Mensch, ATCC HB8065) gezeigt. Da mittlerweile durch Northern blot-Analyse gezeigt werden konnte, daß MP121 in HepG2 (Hepatozytenzellinie aus der Leber) natürlich exprimiert wird, ist davon auszugehen, daß auch der monomeren Form von MP121 eine physiologische Bedeutung zukommen kann. Das monomere MP121 tritt neben dem dimeren MP121 in signifikanten Mengen, z.B. auch auf in Mv1Lu (NBL-7, Lunge, Nerz, ATCC CCL 64) oder Hela (Epitheliales Karzinom, Gebärmutterhals Mensch, ATCC CCL 2).

Bei Expressionsversuchen von MP121 unter Ausnutzung des Baculoviren-Systems in Insektenlarven (Trichoplusia ni) wurde die dimere Form in der Haemolymphe gefunden.

Die Teilreinigung von MP121 aus Zellkulturüberstand erfolgte wie in der WO96/01316 beschrieben über Phenyl-Sepharose und Reversed Phase HPLC. Parallel wurde nach derselben Methodik die entsprechende Menge Zellkulturüberstand nach Infektion mit Wildtyp Viren (wt) als Kontrolle aufgearbeitet. Es konnte bei Versuchen zur Verbesserung der Reinigung gezeigt werden, daß die alternierende Verwendung von Laufmitteln, die TFA (Trifluoressigsäure) oder HFBA (Heptafluorbuttersäure) enthalten kombiniert mit geänderten Gradientenverläufen, der Reinheitsgrad von MP121 wesentlich erhöht werden konnte. Dazu wird z.B. zunächst eine Säule (Aquapore RP-300, Applied Biosystems, Partikelgröße: 7 µm) bei einer Flußgeschwindigkeit von 0,2 ml/min mit 0,1 % TFA (1,36 % Acetonitril pro min), dann eine Säule mit 0,2 % HFBA (0,23 % Acetonitril pro min) und zum Schluß eine Säule noch mal mit 0,1 % TFA (0,23 % Acetonitril pro min) im Laufmittel eluiert. Die eluierten Fraktionen die MP121 enthalten können nach jedem Lauf vereinigt, lyophilisiert und dann für den Auftrag auf die neue Säule in 0,1 % TFA/H₂O resuspendiert werden. Zum Schluß wurden die lyophilisierten Proben bis zum Gebrauch bei -70°C gelagert. Die Mengenabschätzung erfolgte in Western blot-Analysen im Vergleich mit MP121m (Aminosäure 237 bis 352 in SEQ. ID. NO. 2 mit einem zusätzlichen Methionin am N-Terminus), das ähnlich wie MP52, in E.coli, allerdings unter Verwendung des Stammes HMS 174 (DE3) (Novagen #69453) hergestellt wurde. Die Reinigung erfolgte nach Standardmethoden aus Einschlußkörpern mit anschließendem Waschen der Einschlußkörper mit 2 M Guanidiniumchlorid/HCl in 20 mM Tris pH 8.0 (unter Ultraschall) und Resuspension in 6M Guanidiniumchlorid/HCl in 20 mM Tris pH 8,0 mit anschließender Reinigung über die Reversed Phase nach Standardmethoden.

### Beispiel 6:

### Einfluß von MP52 und MP121 auf Neurone der Retina

Um den Einfluß von MP52 und MP121 in einem anderen System zu untersuchen, wurden Gewebekulturen von Retina aus Hühnerembryonen isoliert. Die Methode zur Isolation von ungefähr gleich großen scheibchenförmigen Gewebestückchen aus der Retina ist im Detail beschrieben bei Carri, N.G. und Ebendal, T. (Dev. Brain Res., Vol. 6 (1983), 219-229), Carri, N.G. und Ebendal, T. (Anat. Rec., Vol. 214 (1986), S. 226-229) und Carri, N.G. et al. (J. Neurosci. Res. Vol. 19 (1988), S. 428-439).

In diesem Versuchen mißt man in vitro die Stimulation des Auswachsens von Nervenfasern aus embryonalen Retina Explantaten auf einer Kollagen-Matrix. Kurz beschrieben werden die Gewebeteile mit einer Glaskapillare aus der Retina von Hühnerembryonen (Weißes Leghorn, 6. Tag der Embryonalentwicklung) entnommen und durch wiederholtes Waschen das Pigmentepithel der Retina und mesenchymale Zellen entfernt. Die so behandelten Gewebeteilchen wurden auf Kollagen beschichteten Kulturschalen überführt und über Nacht inkubiert (37,5°C, 5 % CO₂). Anschließend werden die entsprechenden Faktoren oder Kontrollen zugesetzt und die Kulturen weiter inkubiert. Für MP52 wurde das dimere MP52m, so wie es in Beispiel 2 beschrieben ist, in verschiedenen Konzentrationen eingesetzt. Das monomere MP52, das bislang in keinem Versuch Aktivität gezeigt hat, wurde als negative Kontrolle in den entsprechenden Konzentrationen eingesetzt. Für MP121 wurde das in Beispiel 5 beschriebene teilgereinigte Material nach Phenyl-Sepharose und Reversed Phase HPLC in verschiedenen Konzentrationen eingesetzt. Das nach Wildtyp-Infektion entsprechend gewonnene Material wurde als Kontrolle eingesetzt. Als unabhängige Kontrolle wurden die Explantate in Kulturmedium mit etwas Rinderserumalbumin gehalten.

Die Proteine wurden in einem wäßrigen Puffer oder 50 % Acetonitril gelöst und im Kulturmedium auf Endkonzentrationen von 1,25 ng/ml, 12,5 ng/ml, 25 ng/ml, 50 ng/ml, 100 ng/ml, 200 ng/ml weiterverdünnt. Die Art des Lösens hatte keinen Einfluß auf das Ergebnis.

Nach vier Tagen in Kultur wurde die maximale Länge der führenden Nervenbündel unter dem Mikroskop im Dunkelfeld gemessen. Wie in Tabelle 1 und 2 gezeigt, stimulieren sowohl MP52 als auch MP121 dosisabhängig das Auswachsen der Neurite. MP52 hat die maximale Aktivität im Bereich von 25 - 100 ng/ml und MP121 zeigt maximale Aktivität im Bereich von ca. 25 ng/ml, was einer tatsächlichen Faserlänge von 1,3 - 1,7 mm bzw. 1,7 mm gegenüber 0,2 mm für die Negativ-Kontrolle entspricht. Monomeres MP52 und die Wildtyp-Kontrolle zeigten keine Stimulation, die über die Negativ-Konrolle hinaus ging.

**Tabelle 1**

| **dimeres MP52m (ng/ml)** | **Länge (Einheiten)** | **Mittelwert ± SEM** |
|---|---|---|
| 1,25 | 15/9/4/13 | 10,2 ± 2,4 |
| 12,5 | 25/18/10/21 | 18,5 ± 3,1 |
| 25 | 74/38/48/47/27/30 | 44,0 ± 6,9 |
| 50 | 62/65/52/51/50/62 | 57,0 ± 2,4 |
| 100 | 26/33/61/70/57/61 | 51,5 ± 7,3 |
| 200 | 10/13/11/9 | 10,7 ± 0,8 |

- Tabelle 1:: Länge der retinalen Neurite nach 4 Tagen Kultur unter Einwirkung verschiedener Konzentrationen von MP52. Die Neuritlängen der Kontrollen, die nur Kulturmedium enthielten, betrugen 5,5/8/10/11/4,8/7 Einheiten mit einem Mittelwert von 7,7 Einheiten (SEM 1,0). Die Neuritlängen der Kontrollen mit monomeren MP52m (gleiche Konzentrationen wie für dimeres MP52m) ergaben im Mittel gleiche Längen wie die Kontrolle, die nur Kulturmedium enthielt. Eine Einheit entspricht einem tatsächlichen Maßstab von 0,03 mm in der Kulturschale.

**Tabelle 2**

| **MP121 (ng/ml)** | **Länge (Einheiten)** | **Mittelwert ± SEM** |
|---|---|---|
| 1,25 | 7/12/5/6 | 7,5 ± 1,5 |
| 12,5 | 19/20/13/26 | 19,5 ± 2,6 |
| 25 | 50/52/60/71/65/53 | 58,5 ± 3,4 |
| 50 | 37/32/48/41/36/20 | 35,6 ± 3,8 |
| 100 | 21/8/19/18 | 16,5 ± 2,9 |
| 200 | 11/8/12/10 | 10,2 ± 0,8 |

- Tabelle 2:: Länge der retinalen Neurite nach 4 Tagen Kultur unter Einwirkung verschiedener Konzentrationen von MP121. Die Neuritlängen der Kontrolle mit parallel gereinigtem Zellkulturüberstand aus der Wildtyp-Infektion ergaben im Mittel gleiche Längen wie die Kontrolle, die nur Kulturmedium enthielt (siehe Text zu Tabelle 1).Eine Einheit entspricht einem tatsächlichen Maßstab von 0,03 mm in der Kulturschale.

Detaillierte Beschreibung der Figuren:
Figur 1: Silbergefärbtes 15%iges Polyacrylamidgel mit je 0,2 µg MP52His (Spur 2 und 4) oder 0,2 µg MP52m (Spur 3 und 5) nach Aufreinigung der monomeren Form (Spur 2 und 3) bzw. nach dem Zurückfalten zum dimeren aktiven Protein und Abtrennung restlicher Monomere über die HPLC (Spur 4 und 5). Der Molekulargewichtsmarker (15 kD, 25 kD, 35 kD, 50 kD, 75 kD, 100 kD, 150 kD) in Spur 1 ist von Novagen (#69149-1).
Figur 2: Das Chromatogramm zeigt das Laufverhalten von solubilisiertem monomerem MP52His (···) sowie die Trennung von dimerem MP52His von restlichen monomeren Formen nach dem Renaturieren (-) auf einer Reversed Phase HPLC. Dimeres MP52His eluiert unter den gewählten Bedingungen früher als die monomere Form. Der Acetonitrilgradient bezogen auf % Puffer B ist ebenfalls eingezeichnet.
Figur 3: Anzahl der überlebenden TH-immunreaktiven dopaminergen Neurone nach Isolation aus dem Mittelhirn von Rattenembryonen (E14) und 8 Tagen Kultivierung. Neben der Kontrolle mit unbehandelten Neuronen (Medium mit 0.3 % Acetonitril) ist der Effekt bei Zusatz von 20 ng/ml gereinigtem monomerem MP52His aus der Expression in E.coli (MP52His Monomer), 20 ng/ml gereinigtem MP52His aus der Expression in E.coli nach der Renaturierung zum dimeren Protein (MP52His Dimer), sowie 20 ng/ml bzw. 2 ng/ml teilgereinigtem MP52 aus der Expression in Vaccinia Viren (MP52) abgebildet. Gezeigt ist der Mittelwert ± SEM aus einer Dreifachbestimmung.
Figur 4: Fotografie von Zellen nach Isolation aus dem Mittelhirn von Rattenembryonen (E14) nach 8 Tagen Kultur und Anfärben von GFAPs (glial fibrillary acidic proteins). Gezeigt ist der Effekt von 20 ng/ml gereinigtem monomerem MP52His (A) aus der Expression in E.coli, 20 ng/ml gereinigtem dimerem MP52His (B) aus der Expression in E.coli nach der Renaturierung, unbehandelte Zellen (C, Medium mit 0,3 % Acetonitril als Kontrolle), 20 ng/ml (D) bzw. 2 ng/ml (E) teilgereinigtes MP52 aus der Expression in Vaccinia Viren und 2 ng/ml TGF-ß 3 (F).
   Fotografiert wurde jeweils ein repräsentativer Ausschnitt bei 400-facher Vergrößerung im Mikroskop (Axiophot) mit Interferenzkontrast. Die Länge des Balken (-) entspricht 25 µm.
Figur 5: Western blot mit Kaninchen Antikörpern gegen MP121
   1: Zellkulturüberstand von Hep-G2 Zellen nach Infektion mit rekombinanten Vaccinia Viren (mit inserierter MP121 cDNA) unter nicht reduzierenden Bedingungen
   2: Zellkulturüberstand von Hep-G2 Zellen nach Infektion mit Wildtyp Vaccinia Viren unter nicht reduzierenden Bedingungen
   3: vorgefärbter Protein-Molekulargewichtsmarker (schematisch eingezeichnet) mit den apparenten Molekulargewichten von 15,5/18,2/27,8/43,8/71,5 kD (Gibco BRL #26041-020)
   4: Zellkulturüberstand von Hep-G2 Zellen nach Infektion mit rekombinanten Vaccinia Viren (mit inserierter MP121 cDNA) unter reduzierenden Bedingungen
   5: Zellkulturüberstand von Hep-G2 Zellen nach Infektion mit Wildtyp Vaccinia Viren unter reduzierenden Bedingungen
Figur 6: Auswachsen der Nervenfasern aus embryonaler Hühner Retina nach 4 Tagen in Gewebekultur. Mikroskopische Aufnahme von lebenden Kulturen im Dunkelfeld.
   A: monomeres MP52m (5 ng/ml) gereinigt aus Einschlußkörpern von E.coli.
   B: dimeres MP52m (5 ng/ml) gereinigt aus Einschlußkörpern von E.coli und zum nativen dimeren Protein zurückgefalten.
   C: MP121 (5 ng/ml) gereinigt aus Zellkulturüberstand nach Expression mit Hilfe des Vaccinia-Systems auf NIH3T3-Zellen.

## Patentansprüche

1. Verwendung von biologisch aktivem MP52 zur Herstellung eines Arzneimittels zur Behandlung oder/und Prävention von Störungen oder Verlusten nervöser Funktionen, hervorgerufen durch akute oder chronische pathologische Zustände oder/und zur Behandlung neuropathologischer Situationen, die durch Alterung des Nervensystems verursacht sind, wobei als biologisch aktives MP52
(a) der reife Anteil und gegebenenfalls weitere funktionelle Anteile der in SEQ ID NO.1 gezeigten Proteinsequenz;
(b) Teile des reifen Proteins, die dieselbe Aktivität aufweisen;
(c) reife Proteine mit verändertem N-Terminus, welche dieselbe Aktivität aufweisen;
(d) ein reifes Protein oder Teile davon, welches aufgrund seiner Abstammung aus anderen Wirbeltieren Abweichung in der Aminosäuresequenz aufweist, jedoch dieselbe Aktivität beibehält, verwendet wird.

2. Verwendung nach Anspruch 1 zur Herstellung eines Arzneimittels zur Behandlung oder Prävention von Erkrankungen am Auge, insbesondere der neuronalen Schicht der Retina, der Hornhaut, des Sehnervs oder/und anderer Himnerven und zur Verwendung bei Netzhaut- oder Hornhauttransplantationen.

3. Verwendung nach Anspruch 1 zur Herstellung eines Arzneimittels zur Behandlung von akuten pathologischen Zuständen, welche durch Verletzungen hervorgerufen wurden.

4. Verwendung nach Anspruch 1 zur Herstellung eines Arzneimittels zur Behandlung von akuten pathologischen Zuständen, hervorgerufen durch zerebrovaskuläre, entzündliche, infektiöse, stoffwechselartige Mängel und/oder toxische Einflüsse, Tumorwachstum oder operative Eingriffe.

5. Verwendung nach Anspruch 1 zur Herstellung eines Arzneimittels zur Behandlung von neurodegenerativen Erkrankungen.

6. Verwendung nach Anspruch 1 zur Herstellung eines Arzneimittels zur Behandlung von Parkinson'scher Krankheit, Alzheimer oder Huntington Chorea.

7. Verwendung nach einem der Ansprüche 1 bis 6,
**dadurch gekennzeichnet,**
**daß** man ein Fusionsprotein aus biologisch aktivem MP52 oder Teilen hiervon und einem anderen Protein aus der Superfamilie von Proteinen mit "Cystine Knot Motif' oder Teilen hiervon verwendet.

8. Verwendung nach Anspruch 7,
**dadurch gekennzeichnet,**
**daß** man ein Fusionsprotein aus biologisch aktivem MP52 und MP121 oder Teilen hiervon verwendet.

9. Verwendung nach einem der Ansprüche 1 bis 6,
**dadurch gekennzeichnet,**
**daß** man als biologisch aktiven MP52 ein Heterodimer aus einem biologisch aktiven MP52 und einem Monomer eines anderen Proteins aus der Superfamilie der Proteine mit "Cystine Knot Motif" verwendet.

10. Verwendung nach Anspruch 9,
**dadurch gekennzeichnet,**
**daß** man als biologisch aktiven MP52 ein Heterodimer aus einem biologisch aktiven MP52 und MP121 verwendet.

11. Verwendung nach einem der Ansprüche 7 bis 10
zur Herstellung eines Arzneimittels zur Behandlung oder Prävention von Erkrankungen am Auge, insbesondere der neuronalen Schicht der Retina, der Hornhaut, des Sehnervs oder/und anderer Himnerven und zur Verwendung bei Netzhaut- oder Hornhauttransplantationen.

12. Verwendung nach einem der Ansprüche 1 bis 11,
**dadurch gekennzeichnet,**
**daß** man zusätzlich natürlich vorkommende Ganglioside, deren Derivate, Salze oder künstliche Analoga zusetzt.

13. Verwendung nach einem der Ansprüche 1 bis 12,
**dadurch gekennzeichnet,**
**daß** zusätzlich ein Protein aus der Superfamilie der Proteine mit "Cystine Knot Motif" zugesetzt wird.

14. Verwendung nach einem der Ansprüche 7, 9 oder 13,
**dadurch gekennzeichnet,**
**daß** es sich bei dem Protein aus der Superfamilie mit "Cystine Knot Motif" um ein Protein aus der TGF-ß-Superfamilie, NGF-/Neurotrophin-Familie oder PDGF-Familie handelt.

15. Verwendung nach Anspruch 14,
**dadurch gekennzeichnet,**
**daß** es sich um TGF-ß-, BMP-, Aktivin-, GDNF-, NGF-, BDNF-, NT3- oder NT4/5-Proteine handelt.

16. Verwendung nach einem der Ansprüche 1 bis 15,
**dadurch gekennzeichnet,**
**daß** man zusätzlich einen Wachstumsfaktor wie FGF, EGF oder Glial Growth Factor verabreicht.

17. Verwendung nach einem der Ansprüche 1 bis 16,
**dadurch gekennzeichnet,**
**daß** man intrazerebral, oral, durch Injektion, durch Inhalation, als lokale, äußerliche Anwendung verabreicht.

## Claims

1. Use of biologically active MP52 for producing a medicament for treating and/or preventing disorders or losses of nerve function caused by acute or chronic pathological states and/or for treating neuropathological situations which are brought about by ageing of the nervous system, the biologically active MP52 used being
(a) the mature fraction and optionally further functional fractions of the protein sequence shown in SEQ ID no. 1;
(b) parts of the mature protein which exhibit the same activity;
(c) mature proteins with a modified N terminus which exhibit the same activity;
(d) a mature protein or parts thereof which, because it originates from other mammals, differs in the amino acid sequence, but retains the same activity.

2. Use according to claim 1 for producing a medicament for treating or preventing diseases of the eye, in particular of the neural layer of the retina, the cornea, the optic nerve and/or other cranial nerves and for use in retinal or corneal transplants.

3. Use according to claim 1 for producing a medicament for treating acute pathological states which were caused by injuries.

4. Use according to claim 1 for producing a medicament for treating acute pathological states caused by cerebrovascular, inflammatory, infectious, metabolic deficiencies and/or toxic influences, tumour growth or surgical interventions.

5. Use according to claim 1 for producing a medicament for treating neurodegenerative diseases.

6. Use according to claim 1 for producing a medicament for treating Parkinson's, Alzheimer's or Huntington's disease.

7. Use according to any one of claims 1 to 6, **characterised in that** a fusion protein is used which is prepared from biologically active MP52 or parts thereof and another protein from the superfamily of proteins with a "cystine knot motif" or parts thereof.

8. Use according to claim 7, **characterised in that** a fusion protein is used which is prepared from biologically active MP52 and MP121 or parts thereof.

9. Use according to any one of claims 1 to 6, **characterised in that** the biologically active MP52 used is a heterodimer prepared from a biologically active MP52 and a monomer of another protein from the superfamily of proteins with a "cystine knot motif".

10. Use according to claim 9, **characterised in that** the biologically active MP52 used is a heterodimer prepared from a biologically active MP52 and MP121.

11. Use according to any one of claims 7 to 10 for producing a medicament for treating or preventing diseases of the eye, in particular of the neural layer of the retina, the cornea, the optic nerve and/or other cranial nerves and for use in retinal or corneal transplants.

12. Use according to any one of claims 1 to 11, **characterised in that** naturally occurring gangliosides, the derivatives, salts or artificial analogues thereof are additionally added.

13. Use according to any one of claims 1 to 12, **characterised in that** a protein from the superfamily of proteins with a "cystine knot motif" is additionally added.

14. Use according to any one of claims 7, 9 or 13, **characterised in that** the protein from the superfamily with a "cystine knot motif" is a protein from the TGFβ superfamily, NGF/neurotrophin family or PDGF family.

15. Use according to claim 14, **characterised in that** the proteins are TGF-β, BMP, activin, GDNF, NGF, BDNF, NT3 or NT4/5 proteins.

16. Use according to any one of claims 1 to 15, **characterised in that** a growth factor such as FGF, EGF or glial growth factor is additionally administered.

17. Use according to any one of claims 1 to 16, **characterised in that** administration proceeds intracerebrally, orally, by injection, by inhalation, or as topical, external application.

## Revendications

1. Utilisation de MP52 biologiquement actif pour la préparation d'un agent pharmaceutique pour le traitement et/ou la prévention de troubles ou de pertes de fonctions nerveuses, engendrés par des états pathologiques sévères ou chroniques et/ou pour le traitement d'états neuropathologiques, qui sont engendrés par le vieillissement du système nerveux, où comme MP52 biologiquement actif, on utilise
(a) la partie mature et le cas échéant, la partie plus fonctionnelle de la séquence protéique montrée en SEQ ID N°1;
(b) des parties de la protéine mature, qui présentent la même activité ;
(c) des protéines matures avec extrémité N modifiée, qui présentent la même activité ;
(d) une protéine mature ou une partie de celle-ci, qui sur base de son origine d'autres vertébrés, présente une divergence dans la séquence des acides aminés, mais conserve la même activité.

2. Utilisation selon la revendication 1, pour la préparation d'un agent pharmaceutique pour le traitement ou la prévention de maladies des yeux, en particulier de la couche neuronale de la rétine, de la cornée, du nerf optique et/ou d'autres nerfs crâniens, et pour une utilisation lors de la transplantation de rétine ou de cornée.

3. Utilisation selon la revendication 1, pour la préparation d'un agent pharmaceutique pour le traitement d'états pathologiques sévères, qui sont engendrés par des lésions.

4. Utilisation selon la revendication 1, pour la préparation d'un agent pharmaceutique pour le traitement d'états pathologiques sévères, engendrés par des manques cérébrovasculaires, inflammatoires, infectieux ou de type métabolique et/ou des influences toxiques, une croissance tumorale ou des interventions opératoires.

5. Utilisation selon la revendication 1, pour la préparation d'un agent pharmaceutique pour le traitement de maladies neurodégénératives.

6. Utilisation selon la revendication 1, pour la préparation d'un agent pharmaceutique pour le traitement de la maladie de Parkinson, la maladie d'Alzheimer ou la chorée de Huntington.

7. Utilisation selon l'une des revendications 1 à 6, **caractérisée en ce que** l'on utilise une protéine de fusion du MP52 biologiquement actif ou de parties de celui-ci et d'une autre protéine de la superfamille des protéines avec « motif noeud à cystéine » ou des parties de celles-ci.

8. Utilisation selon la revendication 7, **caractérisée en ce que** l'on utilise une protéine de fusion du MP52 et du MP121 biologiquement actif ou de parties de celle-ci.

9. Utilisation selon l'une des revendications 1 à 6, **caractérisée en ce que** l'on utilise comme MP52 biologiquement actif, un hétérodimère d'un MP52 biologiquement actif et d'un monomère d'une autre protéine de la superfamille des protéines avec « motif noeud à cystéine ».

10. Utilisation selon la revendication 9, **caractérisée en ce que** l'on utilise comme MP52 biologiquement actif, un hétérodimère d'un MP52 biologiquement actif et de MP121.

11. Utilisation selon l'une des revendications 7 à 10, pour la préparation d'un agent actif pour le traitement ou la prévention de maladies des yeux, en particulier de la couche neuronale de la rétine, de la cornée, du nerf optique et/ou d'autres nerfs crâniens, et pour une utilisation lors de transplantation de rétine ou de cornée.

12. Utilisation selon l'une des revendications 1 à 11, **caractérisée en ce que** l'on ajoute en outre, des gangliosides naturels, leurs dérivés, sels ou analogues synthétiques.

13. Utilisation selon l'une des revendications 1 à 12, **caractérisée en ce que** l'on ajoute en outre, une protéine de la superfamille des protéines avec « motif noeud à cystéine ».

14. Utilisation selon l'une des revendications 7, 9 ou 13, **caractérisée en ce que** la protéine de la superfamille des protéines avec « motif noeud à cystéine » consiste en une protéine de la superfamille du TGF-β, de la famille des NGF/neurotrophine ou de la famille des PDGF.

15. Utilisation selon la revendication 14, **caractérisée en ce qu'**il s'agit de la protéine TGF-β, BMP, activine, GDNF, NGF, BDNF, NT3 ou NT4/5.

16. Utilisation selon l'une des revendications 1 à 15, **caractérisée en ce que** l'on administre en outre, un facteur de croissance comme FGF, EGF ou le facteur de croissance gliale.

17. Utilisation selon l'une des revendications 1 à 16, **caractérisée en ce que** l'on administre de manière intracérébrale, orale, par injection, par inhalation, par application locale externe.
